# EUROPEAN PATENT APPLICATION

(11) **EP 2 293 072 A1**
(43) Date of publication of application: **09.03.2011**
(21) Application number: 09290659.3
(22) Date of filing: 31.08.2009
(51) Int. Cl.: G01N 33/68, G01N 33/573, C12Q 1/37

(54) **Use of cathepsin H**

(71) Applicant: Sanofi-Aventis, 75013 Paris (FR)
(72) Inventor: The designation of the inventor has not yet been filed
(74) Representative: Körner, Kathrin

(57) **Abstract**

Present invention concerns the use of Cathepsin H. Other aspects of the invention concern methods for screening pharmaceuticals, for diagnosing pain susceptibility and for the treatment of pain.

## Description

Present invention concerns the use of Cathepsin H. Other aspects of the invention concern methods for screening pharmaceuticals, for diagnosing pain susceptibility and for the treatment of pain.

In the western world, chronic pain is a major unsolved health problem undermining the health and welfare of millions of citizens. Chronic pain severely afflicts the well-being of the individual experiencing it and it is frequently accompanied or followed by vegetative signs, which often result in depression. Chronic pain results in individual suffering and social economic costs of tremendous extent. Existing pharmacological pain therapies are widely unsatisfying both in terms of efficacy and of safety.

In light of the severe drawbacks connected with state of the art pain treatments, there is a great need for novel options for treatment of ongoing pain, and for diagnosis and prognosis concerning the potential development of chronic pain. Especially in light of the vast gap between the fast advancing understanding of the neurobiology of pain and the unmet clinical need to provide effective treatments without the drawbacks of state of the art treatments, efforts need to be directed to the discovery of new targets for novel classes of analgesics.

Thus, it is the object of the present invention to provide a new means for the development and provision of new classes of pain modulating drugs.

This object is solved by the use of Cathepsin H or functional fragments or derivatives thereof for identifying compounds that modulate pain and especially neuropathic pain.

The invention is based on the surprising finding of the inventors, demonstrating for the first time that Cathepsin H expression closely correlates with pain susceptibility in mouse models of neuropathic pain.

Pain is, per definition of the international association for the study of pain, an unpleasant sensory and emotional experience associated with actual or potential tissue damage, or described in terms of such damage. Pain is normally the consequence of an activation of the nociceptive nervous sytem, that is specialized to detect and encode damage or potential damage of tissue. Pain is thus part of a warning system of the body to initiate reactions for minimizing actual or potential damage to the body. Pain can be the primary symptom of a medical condition or can be secondary effect of a diseased state, often without any biological meaning.

Pain may be acute or chronic. Acute pain is a physiological signal indicating a potential or actual injury. It occurs accompanying tissue damage, infection, inflammation or other acute causes, alerting the individual after bodily damage or malfunction. If acute pain is not treated properly, it may lead to chronic pain.

Chronic pain is a diseased state with varying origin, duration, intensity and specific symptoms.

Chronic pain may be of nociceptive origin, inflammatory or neuropathic. Nociceptive pain is judged to be commensurate with ongoing activation of somatic or visceral pain-sensitive nerve fibers. Neuropathic pain is pain resulting from any kind of damage to peripheral or central neuronal tissue; it is believed to be sustained by aberrant somatosensory processes in the peripheral nervous system, the CNS, or both. (For an overview of pain mechanisms, see for example Scholz and Woolf, 2002; Julius and Basbaum. 2001, Woolf and Mannion, 1999; Wood, J.D., 2000; Woolf and Salter, 2000.)

Chronic neuropathic pain is variable from patient to patient. Recent data indicate that individual pain susceptibility plays an important role for the amount of individual suffering, i.e. there is an important heritable predisposition to pain, particularly to the development of neuropathic pain. Present invention is based on extensive studies of the inventors which aimed to identify pain susceptibility genes (i.e. genes that determine the amount of pain felt in the presence of a given, fixed degree of tissue injury) in rodent models of chronic pain. The rodent models and experimental settings used by the inventors allowed for experimental conditions where among the different individuals a) nature and uniformity of the neural lesion can be precisely controlled and b) genetic and environmental variability can be minimized.

Cathepsin H (CTSH; alternative titles according to the Atlas of Genetics and Cytogenetics in Oncology and Haematology (http://atlasgeneticsoncology.org): ACC-4, ACC-5, CPSB, DKFZp686B24257, EC 3.4.22.16, MGC1519, aleurain, minichain) is a lysosomal protease.

The gene locus of human Cathepsin H is on chromosome 15q24-125 (see e.g. Atlas of Genetics and Cytogenetics in Oncology and Haematology). The gene contains 12 exons spanning over 23 kb genomic sequences. The nucleotide sequence of the human preprocathepsin H was e.g. published by Fuchs and Gassen, 1989, Nucleic Acids Res. 17: 9471-9471. The gene structure of rat cathepsin H was published e.g. by Ishidoh et al, FEBS Letters, 1989, Vol. 253, number 1,2, 103-107. The genomic sequence of homo sapiens CTSH on chromosome 15 can e.g. be retrieved at the NCBI homepage under accession number NG_009614.1 (SEQ ID NO.1).

The gene of Cathepsin H has a TATA- and CAAT-less promoter and upstream of exon 1 one GC bo. Two different forms of cathepsin H cDNA have been detected in prostate tissues and cancer cell lines: a full-length form (CTSH) and a truncated form with deletion of 12 amino acids at the signal peptide region (CTSHdelta 10-21) (see Atlas of Genetics and Cytogenetics in Oncology and Haematology). The length of the preproenzyme transcript is 1005 bp. The coding polynucleotide sequence of Cathepsin H is publicly available at the NCBI nucleotide database under several accession numbers, such as: BC006878 (CTSH, complete coding sequence, mus musculus, SEQ ID NO.4, NM_004390.3 (Homo sapiens CTSH transcript variant1 mRNA (SEQ ID NO.2), NM_148979.2 (Homo sapiens CTSH, transcript variant 2 mRNA (SEQ ID NO.3)),. The skilled person knows how to retrieve further coding or genomic sequences of Cathepsin H from the NCBI database (Cathepsin H of other species; mutants or different isoforms of Cathepsin H, if existing). If in the following, it is referred to the Cathepsin H coding sequence, this can mean any of the above mRNA or coding sequences; with sequences according to SEQ ID. NOs. 2, 3 and 4 being preferred examples.

The protein sequence of Cathepsin H is publicly available at the NCBI protein database, e.g. under the following accession numbers: homo sapiens (hs) Cathepsin H isoform a preproprotein: NP_004381 (SEQ ID No.8); hs isoform b precursor: NP:683880 (SEQ ID NO.9); murine (mus musculus) Cathepsin H: preproprotein: NP_031827; mus musculus isoform CRA_a: EDL20900, mus musculus isoform CRA_b: EDL20901, rat (Rattus norvegicus) Cathepsin H: NP_037071; cathepsin H isoform CRA-a (Rattus norvegicus): EDL77562, isoform CRA-b (Rattus norvegicus) of Cathepsin H: EDL77563, cathepsin H, Moreover the protein sequence is publicly available at the UniProtKB database (www.beta.uniprot.org), under accession numbers: P09668 (HUMAN_CATH, human Cathepsin H, SEQ ID NO.5) If in the following, it is referred to the Cathepsin H protein or amino acid sequence, this can mean any of the above protein sequences or translated protein sequences from the above listed coding sequences; such as the following sequences: SEQ ID NO.s 5, 6, 7, 8 or 9.

NCBI is the national centre for biotechnology information (postal address: National Centre for Biotechnology Information, National Library of Medicine, Building 38A, Bethesda, MD 20894, USA; web-adress: www.ncbi.nhm.nih.gov). More sequences (e.g. sequences carrying SwissProt or EMBL accession numbers) can be retrieved in the UniProtKB database under www.beta.uniprot.org.

Cathepsin H is a lysosomal protease with aminopeptidase and weaker endopeptidase function. It belongs to the family of C1 (papain-like) cystein proteases. Cathepsin H protein is synthesized as a preproenzyme of 335 amino acids and proteolytically processed into an active single chain inside of endosomes or lysosomes. In addition to the heavy and light chain (together denominated long chain), Cathepsin H contains a so-called mini chain "EPQNCSAT" that is derived from the propeptide. (For the structure of Cathepsin H, see also Turk et al., Biological Chemistry, 1997). The mini chain appears to be involved in the aminipeptidase activity of Cathepsin H and play a key role in substrate recognition. A recombinant form of human Cathepsin H lacking the mini-chain was shown to be an endopeptidase (Valiljeva et al, 2003). Endopeptidase substrates of Cathepsin H are e.g.: Bz-Arg+NHNap, Bz-Arg+NH-Mec, Bz-Phe-Val-Arg+NHMec,. It has dipeptidyl-peptidase activity for the substrates: Pro-Gly+Phe and Pro-Arg+NHNap. It has aminopeptidase activity for the substrates: H-Arg-NH-Mec, Bz-Arg-NH-Mec, Bz-Arg-NH-Mec and H-Cit-NH-Mec (see e.g. Rothe and Dodt, 1992; Bz=benzoyl, NH-Mec = e-methylcoumaryl-7-amide; Cit= citrulline). Naturally occurring inhibitors of Cathepsin H are the cystatins, alpha2-macroglobulin as well as antigens from mouse cytotoxic lymphocytes CTLA-2beta.

Cathepsin H is ubiquitiously expressed with highest level in the kidney. Its expression may be increased in some cancerous tissues. Cathepsin is predominantly located in the endosomal-lysosomal compartment, but also secreted to some extent and circulating in the blood.

The functions of Cathepsin H comprise in general: protease activity, e.g hydrolase activity, peptidase activity, e.g. aminopeptidase, dipeptidylpeptidase, exopeptidase or endopeptidase activity, transacylase activity (see Koga et al., 1991); cleavage of the above-listed substrates; cleavage of native C5 and generation of chamotaxin C5a, processing of hydrophobic surfactant-associated protein C, cleavage and/or degradation of fibronectin and fibrinogen. More specifically, it is involved as a lysosomal cystein protease in the intracellular protein degradation. It is involved in the generation of chemotaxin C5a by cleavage of native C5. Cathepsin H is involved in the processing of hydrophobic surfactant-associated protein C. It furthermore appears to be involved in the development of unstable atherosclerotic plaques, and possibly also in early atherogenesis.

The use according to present invention allows for the identification of novel substances for the prevention and/or treatment of pain and especially of neuropathic pain. The use according to present invention comprises the identification of compounds with desired characteristics (i.e. lowering the pain sensation) as well as the identification of compounds with undesired characteristics (i.e. increasing the pain sensation). Moreover, present invention allows for the further characterisation of compounds already identified of being useful for the prevention and/or treatment of any disease or diseased state. In this case, present invention can e.g. be used for excluding identified active compounds having unwanted side-effects (i.e. the increase of pain sensation): Candidate compounds for a given disease can e.g. be profiled for their influence on Cathepsin H (protein and/or nucleic acid, expression and/or function, etc.).

A compound / test compound / active compound as to be employed for the different aspects of present invention can be any biological or chemical substance or natural product extract, either purified, partially purified, synthesized or manufactured by means of biochemical or molecular biological methods.

A compound considered as being active in modulating pain in the sense of the different aspects of present invention can be any substance having an influence on one of the functions of Cathepsin H or on the Cathepsin H amount (protein or nucleic acid) in a cell, on Cathepsin H expression, posttranslational modification (e.g. N-glycosilation or processing (e.g. cleavage), protein folding or activation.

To this end, the substance can modulate any of the functions of Cathepsin H (e.g. those as listed above or below). Cathepsin H protein activity can be modulated by the substance e.g by direct interaction and interference of Cathepsin H polypeptide/protein or fragments thereof. The substance can also modulate Cathepsin H expression, e.g. on the level of transcription (initiation, elongation, processing, etc), transcript stability, translation. Moreover it can modulate the posttranslational modification, the processing from the inactive to the active form (cleavage of the prepro-form to the the active protein), as well as protein folding etc. of Cathepsin H. The substance can exert the above effects directly or indirectly (indirectly meaning i.e. by interfering (positively or negatively) with natural signalling cascades having influence on Cathepsin H function / protein activity / expression etc.)

Functions of Cathepsin H comprise those as listed above, e.g. protease activity; the ability of removing dipeptides from the amino terminus of one or more protein substrates; the ability to interact specifically with one or more protein substrates (protein-protein interaction), such as those listed above; the ability to cleave and/or activate one or more protein substrates, such as those listed above; the ability to process protein or peptide substrates, such those listed above, the ability to be inhibited by one of the inhibitors as listed above.

Functions of Cathepsin H comprise also generally the ability of Cathepsin H protein or nucleic acid or fragments thereof to interact with other molecules (comprising, but not limited to: proteins, peptides, nucleic acids, synthetic molecules) and preferably concern its capability of interacting and cleaving protein substrates.

Substrate of an enzyme is understood, within the terms of present application, to be any molecule that can be modified by the enzyme. Naturally occurring substrates in the scope of present invention are molecules that correspond to the form in which they occur in the natural physiological or pathological context (such as those listed above), and which are also capable of being modified by the respective enzyme.

The modulation of pain and especially neuropathic pain can be either a decrease or an increase.

According to one aspect of present group of inter-related inventions, a fragment or derivative of Cathepsin H can be used. A fragment can be a fragment of a protein, polypeptide or polynucleic acid.

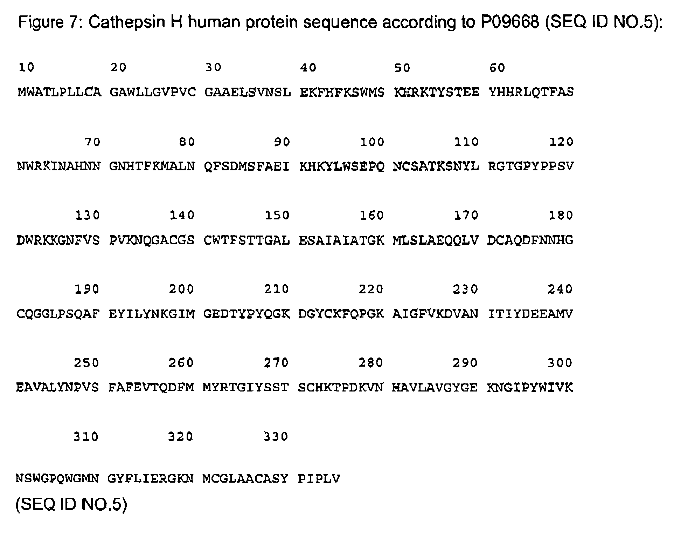

A fragment of a protein or polypeptide is a protein or polypeptide that carries one or more end-terminal (n- and/or c-terminal) and/or internal deletions of one, two or more amino acids, when compared to the full-length Examples of Cathepsin H; fragments comprise, e.g. the domains and/or fragments as listed in the description of Figure 7 (see below).

A functional fragment of Cathepsin H protein is any fragment of this protein having at least one or more of the functional characteristics of the full-length protein, especially as listed above.

A fragment of a polynucleotide acid is a polynucleotide acid or an oligonucleotide carrying one or more end-terminal (5'- and/or 3'-) and/or internal deletions of one, two or more nucleotides, when compared to the full-length genomic or coding sequence. A functional fragment of Cathepsin H nucleic acid is any fragment having at least one or more of the functional characteristics of the full-length polynucleic acid (mRNA, genomic or coding sequence).

The term derivative of Cathepsin H comprises any type of modification of Cathepsin H in comparison to the naturally-occurring form, and especially in comparison to Cathepsin H according to SEQ IDs NO: 5, 6, 7, 8 or 9, that is not a deletion. A functional derivative of Cathepsin H is any derivative of this protein having at least one and preferably two or more of the functional characteristics of the unmodified protein. Derivatives comprise, e.g. modifications of the amino acid or nucleotide sequence or any other kind of modification such as a chemical or biological modification leading e.g. to the stabilization of the polypeptide or polynucleotide (such as phosphoorothioate modifications of the nucleic acid backbone or of exchanges of the bonds between amino acids, etc), or enabling a specific targeting of the plypeptide or polynucleotide to certain cells or facilitating its entry into or its uptake by cells (such as cell-permenat phosphopeptides, ortho coupling to cell-permeant peptide vectors, e.g. based on the antennapedia/penetrating, TAT and signal-peptide based sequences; or coupling to parts of ligands for specific transporters or importers).

Another aspect of present invention concerns the use of a non-human transgenic animal heterologously expressing Cathepsin H or a functional fragment thereof for identifying or analyzing compounds that modulate pain and especially neuropathic pain.

The non human animal can be any non human animal. Preferred are rodents, such as rats or mice.

A transgenic animal is an animal that carries in its genome foreign DNA, which has deliberately been transferred thereto. The introduction of the foreign DNA into the animal genome can be performed according to standard procedures (see e.g. Transgenic Animal Technology A Laboratory Handboook. C.A. Pinkert, editor; Academic Press Inc., San Diego, California, 1994 (ISBN: 0125571658).

The term heterologous expression refers to an expression differing from the normal gene expression in the the host organism (concerning steady state level, amount, timing or tissue distribution of the expressed gene or concerning the type of expressed gene (i.e. the gene is normally not expressed in the host at all)). The heterologous expression can be constitutive or inducible. Suitable inducible expression systems are well known in the art (e.g. the Tetracycline inducible system or the like). The organism can be a cell or a non-human animal.

According to another aspect, present invention concerns the use of a non-human transgenic animal heterologously expressing Cathepsin H or a functional fragment thereof as a model system for enhanced pain sensitivity, especially neuropathic pain sensitivity.

Yet another aspect of present invention concerns the use of a non-human Cathepsin H knock-out animal for identifying or analyzing compounds that modulate pain and especially neuropathic pain.

A knock-out organism (such as an animal or a cell) refers to an organism in which the expression or function of a gene is partially or completely deleted and comprises genomic as well as functional knock outs, inducible as well as constitutive knock outs. The generation of knock out organisms is well known in the art, as well as cells or animals which can be used for generating knock out organisms. The generation of Cathepsin H - knock out mice is described in Pham and Ley, 1999.

A further aspect of present invention concerns use of a non-human Cathepsin H knock-out animal as a model system for lowered pain and especially lowered neuropathic pain sensitivity.

The use of a cell heterologously expressing Cathepsin H or a functional fragment thereof for identifying compounds that modulate pain, especially neuropathic pain, is another aspect of present invention.

The cell can be any prokaryotic or eucaryotic cell, such as cells capable of being transfected with a nucleic acid vector and of expressing a reporter gene. These comprise principally primary cells and cells from a cell culture, such as a eukaryotic cell culture comprising cells derived either from multicellular organisms and tissue (such as HeLA, CHO, COS, SF9 or 3T3 cells) or single cell organisms such as yeast (e.g. s. pombe or s. cerevisiae), or a procaryotic cell culture, preferably Pichia or E.coli. Cells and samples derived from tissue can be gained by well-known techniques, such as taking of blood, tissue punction or surgical techniques.

According to one embodiment, a modified cell, having a lower Cathepsin H activity as compared to its unmodified state, is used. This way, it can e.g, be tested, if the compounds to be tested are able to enhance or restore the lowered or totally abolished Cathepsin H activity. Or it can be tested whether the substances are able to perform their function (e.g. pain modulation or even a function in the context of another diseased state or disease) in the context of lowered pain sensitivity.

The modification can be any type of modification (stable or transient, preferably stable), that leads to a decrease of Cathepsin H activity , Cathepsin H transcript steady state level (i.e. by activation of Cathepsin H transcription or transcript stabilisation) or Cathepsin H protein steady state level (i.e. by activation of Cathepsin H translation or its posttranslational processing; by modulation of Cathepsin H posttranslational modification or by activation of its stabilisation or by inhibition of its degradation). This can for example be achieved by using dominant negative mutants of Cathepsin H, antisense oligonucleotides, RNAi constructs of Cathepsin H, by generating functional or genomic Cathepsin H knock outs (which can e.g. be inducible) or other suitable techniques known within the state of the art. For an overview of the above techniques, see for example: Current protocols in Molecular biology (2000) J.G. Seidman, Chapter 23, Supplemtent 52, John Wiley and Sons, Inc.; Gene Targeting: a practical approach (1995), Editor: A.L. Joyner, IRL Press; Genetic Manipulation of Receptor Expression and Function, 2000; Antisense Therapeutics, 1996; Scherr et al, 2003.

According to one embodiment, a Cathepsin H knock-out cell is used. Suitable cell lines for the generation of knock-outs are well known in the state of the art, see e.g., Current protocols in Molecular Biology (2000) J.G. Seidman, Chapter 23, Supplement 52, John Wiley and Sons, Inc; or Gene Targeting a practical approach. (1995) Ed. A.L. Joyner, IRL Press. The generation of Cathepsin H (DPPI) knock-out cells is also published in Pham and Ley, 1999 (the generation of DPPI murine embryonic stem cell knock out clones, see page 8628, left column, upper half).

Another aspect of the invention concerns thus the use of a Cathepsin knock-out cell for identifying or analyzing compounds that modulate pain, especially neuropathic pain.

Furthermore, the use of a Cathepsin knock-out cell as a model system for lowered pain, especially lowered neuropathic pain sensitivity, is another aspect of present group of inter-related inventions.

According to another embodiment of present invention, the cell can have a higher amount of Cathepsin H as compared to a reference cell (e.g. the same cell in its unmodified state). This cellular system can serve to mimick a state of enhanced pain sensitivity, as the amount of Cathepsin H expression is related to pain sensitivity.

Present invention relates thus also to the use of a cell heterologously expressing Cathepsin H or a functional fragment thereof as a model system for enhanced pain sensitivity, especially neuropathic pain sensitivity.

The use of a cell heterologously expressing a reporter gene expressibly linked to the Cathepsin H promoter and/or enhancer for identifying or analyzing compounds that modulate pain, especially neuropathic pain, is another embodiment of present set of related inventions.

The above aspect of present invention is based on a typical reporter gene assay commonly known in the art. To this end, the promoter of choice is inserted into an expression vector suitable for the type of host cell chosen, upstream of the reporter gene of choice in such a way as to allow for an expression of the reporter gene if the promoter is active. The construct is subsequently introduced into the host cell of choice. Suitable methods for transformation or transfection are well known in the art as well as conditions for cell cultivation and detection of reporter gene expression (see e.g. standard literature listed below). Suitable conditions are well known in the art as well as vectors, reporter genes and necessary reagents, which are also commercially available.

A vector is a circular or linear polynucleotide molecule, e.g. a DNA plasmid, bacteriophage or cosmid, by aid of which polynucleotide fragments (e.g. cut out from other vectors or amplified by PCR and inserted in the cloning vector) can specifically be amplified in suitable cells or organisms. Expression vectors enable the heterologous expression of a gene of interest (e.g. a reporter gene), in the host cell or organism. The type of cell or organism largely depends on the aim and the choice lies within the knowledge of the skilled artisan. Suitable organisms for the amplification of a nucleic acid are e.g. mostly single cell organisms with high proliferation rates, like e.g. bacteria or yeast. Suitable organisms can also be cells isolated and cultivated from multicellular tissues, like e.g. cell lines generated from diverse organisms (e.g. SF9 cells from Spodoptera Frugiperda, etc.). Suitable cloning vectors are known in the art and commercially available at diverse biotech suppliers like, e.g. Roche Diagnostics, New England Biolabs, Promega, Stratagene and many more. Suitable cell lines are e.g. commercially available at the American Type Culture Collection (ATCC).

For the heterologous expression of a protein or polypeptide, the cell can be any prokaryotic or eucaryotic cell capable of being transfected with a nucleic acid vector and of expressing the gene of interest, e.g. a reporter gene. These comprise principally primary cells and cells from a cell culture, preferably an eukaryotic cell culture comprising cells derived either from multicellular organisms and tissue (such as HEK293, RIN-5F, HeLA, CHO, COS, SF9 or 3T3 cells) or single cell organisms such as yeast (e.g. S. pombe or S. cerevisiae), or a procaryotic cell culture, preferably Pichia or E. coli. Cells and samples derived from tissue can be gained by well-known techniques, such as taking of blood, tissue punction or surgical techniques.

Within the context of present application, the term "transfection" refers to the introduction of a nucleic acid vector into a host cell (either prokaryotic or eucaryotic) and comprises thus the term "transformation".

The transfection can be a stable or transient transfection.

The Cathepsin H promoter is a part of the Cathepsin H gene able to drive expression of a gene product of interest if introduced into a suitable expression vector upstream of the coding sequence of the gene product. The cathepsin H promoter is part of the genomic upstream of 5'-sequence of the Cathepsin H gene that steers transcriptional activation of the downstream, transcribed region and can e.g. be derived from Ishidoh et al., FEBS letters, 1989 or Ishidoh et al, Biomed. Biochim. Acta, 1991, 50(4): 541-7.

A reporter gene can be any gene that allows for an easy quantification of its gene product. A vast variety of reporter genes for eukaryotic or prokaryotic hosts as well as detection methods and necessary reagents are known in the art and commercially available. These comprise e.g. the genes of beta Lactamase (lacZ), Luciferase, Green or Blue fluorescent protein (GFP or BFP), DsRed, HIS3, URA3, TRP1 or LEU2 or beta Galactosidase. These genes encode proteins which can be easily detected by means of a visible (colour or luminescent) reaction (e.g. lacZ, Luciferase). These comprise gene-products which can be easily detected by means of a visible (colour or luminescent) reaction or gene-products conferring resistance towards antibiotics like Ampicillin or Kanamycin when expressed. Other reporter gene-products enable the expressing cells to grow under certain conditions like e.g. auxotrophic genes.

A functional fragment of a reporter gene is any fragment of a given reporter gene that allows for an easy quantification of its gene product.

A functional fragment of a reporter gene is any fragment of a given reporter gene that allows for an easy quantification of its gene product.

Within the context of present application, the term "transfection" refers to the introduction of a nucleic acid vector into a host cell (either prokaryotic or eucaryotic) and comprises thus the term "transformation".

The transfection can be a stable or transient transfection.

The cell can be any prokaryotic or eucaryotic cell capable of being transfected with a nucleic acid vector and of expressing a reporter gene. These comprise principally primary cells and cells from a cell culture, preferably a eukaryotic cell culture comprising cells derived either from multicellular organisms and tissue (such as HeLA, CHO, COS, SF9 or 3T3 cells) or single cell organisms such as yeast (e.g. s. pombe or s. cerevisiae), or a procaryotic cell culture, preferably Pichia or E.coli. Cells and samples derived from tissue can be gained by well-known techniques, such as taking of blood, tissue punction or surgical techniques.

Within the context of the above aspect of present invention the control vector can be any suitable vector which comprises a reporter gene or functional fragment thereof, but wherein reporter gene expression is not driven by a (functional) Cathepsin H promoter. This can e.g. mean that the reporter gene or functional fragment thereof is not operationally coupled to a functional Cathepsin H promoter (i.e. either totally devoid of a Cathepsin H promotor, comprises a non functional Cathepsin H promoter or promoter fragment or wherein the coupling of promoter and reporter gene is not functional). Another possibility is that the reporter gene or functional fragment thereof is operationally coupled to another promoter than the Cathepsin H promoter (e.g. SV40 or another standard promoter). The functional vector and the control vector can also be transfected to the same cell, but in which case the reporter genes need to be different.

The identification of compounds according to the above uses can e.g. be performed according to assays as described below or as known in the art.

An assay is any type of analytical method or system to monitor a biological process. Suitably, molecular cascades and mechanisms representing parts of physiological metabolic pathways but also of pathological conditions are reproduced in cellular or biochemical (in vitro) systems. The pharmacological activity of a potential pharmaceutical compound can thus be determined according to its capability of interfering with or modulating these cascades or mechanisms.

For the use in drug screening, especially the high throughput screening for novel pharmaceutical compounds, the assay needs to be reproducible and is preferably also scalable and robust. In the scope of present invention, high throughput screen means, that a method according to present invention is performed in a very small scale, e.g. on 96, 386 or 1536 well plates in samples of very small volume in the range of few millilitres down to few nanoliters or even less. Thus, a very large amount of samples can be analysed in a short time. High throughput screening mostly comprises the screening of approximately 500.000 different compounds for a certain ability by means of one single assay. The assay is preferably suitable for high throughput screening of chemical substances for their ability of modulating the activity of the target molecule under investigation. The type of assay depends e.g. on the type of target molecule used (e.g. polypeptide or polynucleotide) and the "read out", i.e. the parameter, according to which the activity of the target molecule is determined (see below).

Different types of such assays are commonly known in the state of the art and commercially available from commercial suppliers.

Suitable assays for different purposes encompass radioisotopic or fluorescent assays, for example fluorescence polarization assays (such as those offered commercially by Panvera) or Packard BioScience (HTRF; ALPHAScreen™) for measuring the interaction of a labeled member with a non-labeled member (e.g. the interaction of labeled proteins with their unlabeled protein-ligands).

More examples include cell based assays, wherein a cell line stably (inducibly or not; chromosomal or episomal) or transiently expresses a recombinant protein of interest. These assays comprise e.g. reporter gene assays, wherein the regulation of a certain promotor or a signal transduction pathway of a member of a signal transduction cascade is measured according to the activity of a reporter enzyme, the expression of which is under the control of said certain promotor. For this type of assay, a recombinant cell line has to be constructed containing the reporter gene under the control of a defined promotor that is to be investigated itself or that is regulated by the signaling cascade under investigation. Suitable reporter enzymes are commonly known within the state of the art and comprise firefly luciferase, renilla luciferase (e.g. commercially available by Packard reagents), β-Galactosidase. Suitable cell lines depend on the aim of the assay but comprise mostly cell lines that are easy to transfect and easy to cultivate, such as, e.g. HeLA, COS, CHO, NIH-3T3, etc.

For determination of protease activity, typical protease assay formats are known: e.g. using a substrate carrying a reporter tag (e.g. a luminescent/fluorescent or other signal emitting protein/peptide or entity) at one position of the substrate and a quencher (an entitiy (e.g. another peptide inhibiting the signal emission of the reporter tag as long as the substrate is intact/uncleaved) at another position; the substrate is incubated with Cathepsin H under suitable conditions to allow for the cleavage of the substrate leading to the emission of a detectable signal (e.g. light-emission), because of the separation of quencher and reporter tag.

Other types of assays and other types of "read out" are well known in the state of the art. One assay for the detection of cathepsin H activity in cells can e.g. be gained from Rüttger et. Al., BioTechniques, 2006.

Assays according to present invention concern e.g.:
A method of identifying or analyzing compounds modulating and/or preventing pain, preferably neuropathic pain, comprising the steps
   a. Providing at least two samples;
   b. Contacting one sample containing Cathepsin H or a functional fragment or derivative thereof with a compound,
   c. determining the activity of Cathepsin H in the presence of compound,
   d. determining the activity of Cathepsin H in the absence of compound, and
   e. comparing the activity of Cathepsin H according to c) with that according to d).
A method for identifying or analyzing compounds that modulate and/or prevent pain, preferably neuropathic pain, comprising:
   a Contacting a Cathepsin H protein or functional fragment or derivative thereof with a test compound; and
   b Determining whether the test compound modulates the activity of the Cathepsin H protein or functional fragment or derivative thereof.
A method for identifying or analyzing compounds that modulate and/or prevent pain, preferably neuropathic pain, comprising:
   a. Contacting a cell, which has a detectable amount or activity of Cathepsin H or of a functional fragment or derivative thereof, with a test compound;
   b. Determining whether the test compound is able to modulate the amount or activity of Cathepsin H or the functional fragment or derivative thereof present in the cell.
A method for identifying or analyzing compounds that modulate and/or prevent pain, preferably neuropathic pain comprising:
   a. Contacting a nucleic acid coding for a Cathepsin H protein or a functional fragment or derivative thereof with a test compound in a transcriptionally active system, and
   b. Determining the amount of mRNA coding for the Cathepsin H protein or the functional fragment or derivative thereof present in said system in presence of said compound, and
   c. Determining whether the compound is capable of modulating the amount of mRNA coding for the Cathepsin H protein or functional fragment or derivative present in said system.

A transcriptionally active system is any biochemical or cellular system which at least has the ability to perform a transcription reaction of a transcription unit. Such systems are well known in the art and comprise cells as well as in vitro transcription systems or kits (e.g. on basis of cell extracts) commercially available.

A method for identifying compounds or analyzing compounds that modulate and/or prevent pain, preferably neuropathic pain, comprising:
a. Providing a cell transfected with a nucleic acid vector comprising the promoter of a Cathepsin H gene or a functional fragment thereof operationally coupled to a reporter gene or a functional fragment thereof:

b. Providing a cell transfected with a control vector which comprises a reporter gene or a functional fragment thereof not being operationally coupled to a functional Cathepsin H promoter;
c. Determining the reporter gene activity of the cell according to a) and b) in the presence of a test compound;
d. Determining the reporter gene activity of the cell according to a) and b) in absence of the test compound.

A method for identifying or analyzing a compound that modulates pain, preferably neuropathic pain comprising
a. Selecting a compound that modulates the activity of Cathepsin H as a test compound, and
b. Administering said test compound to a subject in sensation of pain to determine whether the pain is modulated.

A method of identifying or analyzing a compound that modulates and/or prevents pain, preferably neuropathic pain in a subject comprising:
c. Assaying a biological activity of Cathepsin H or a functional fragment or derivative thereof in the presence of one or more test compounds to identify one or more modulating compounds that modulate the biological activity of Cathepsin H, and
d. Testing one or more of the modulating compounds for their ability to reduce pain (especially neuropathic pain) and/or pain sensation (especially neuropathic pain sensation) and/ or pain sensitivity (especially neuropathic pain sensitivity) in a subject.

Further aspects of present invention concern pharmacogenomic methods for classifying patient groups and assisting the physician to adapt/improve his treatment of individual patients, such as:

A method for analyzing the pain (especially neuropathic pain) threshold in an individual comprising analyzing the amount of Cathepsin H in a taken sample of said individual in comparison to one or more reference samples as to whether the amount of Cathepsin H mRNA and/or protein present in said sample is different from that of one or more reference samples, wherein the presence of a higher amount indicates an increased pain (especially neuropathic pain) sensitivity and the presence of a lower amount indicates a decreased pain (especially neuropathic pain) sensitivity in said individual.

A method for adapting the dosage of a pharmaceutical for the prevention and/or treatment of pain in an individual, which method comprises examining a taken sample of an individual as to whether the amount of Cathepsin H mRNA and/or protein present in said sample is different from that of one or more reference samples, said dosage being adapted depending on whether the amount of protein and/or mRNA in the taken sample of the individual is different from that of the one or more reference samples, wherein a higher amount of Cathepsin H in the taken sample of the individual is indicative of a need for a higher dose and a lower amount of Cathepsin H in the sample of the individual is indicative of a need for a lower dose.

The term "taken sample" as used herein, refers to a biological sample taken/separated from the body of one or more individual beings (humans or non-human animals). Biological material and biological samples comprise, e.g. cells, preparations or parts of tissue or organs (e.g. brain, blood, liver, spleen, kidney, heart, blood vessels, etc.), preferably if derived from a vertebrate, and more preferably from a mammal including a human. Comprised are also cells from a cell culture, preferably a eukaryotic cell culture comprising cells derived either from multicellular organisms and tissue (such as HeLA, CHO, COS, SF9 or 3T3 cells) or single cell organisms such as yeast (e.g. s. pombe or s. cerevisiae), or a procaryotic cell culture, preferably Pichia or E.coli. Cells and samples derived from tissue can be gained by well-known techniques, such as taking of blood, tissue punction or surgical techniques. The preparation of recombinant molecules and the purification of naturally occuring molecules from cells or tissue, as well as the preparation of cell- or tissue extracts is well known to the person of skill in the art (see e.g. also the standard literature listed below).

The term "reference sample" refers to a biological sample taken from one or more individuals with a known given pain phenotype or to an *in vitro* biological sample (e.g. a sample stemming from in vitro cell or tissue culture (e.g. cultivated cells)) and corresponding in certain characteristics (e.g. its level of Cathepsin H activity, amount or expression) to a given pain phenotype (e.g. high pain sensitivity or low pain sensitivity).

Yet another aspect of present invention concerns the use of a means for the detection of Cathepsin H for determining enhanced pain sensitivity (especially neuropathic pain sensitivity) in an individual by analyzing a biological sample taken from the body of an individual to be examined.

The means for the detection of Cathepsin H can be any means able to specifically detect Cathepsin H polypeptide/protein or nucleic acid present in a biological sample.

A means to detect Cathepsin H protein or polypeptide can be any means able to specifically detect either wildtype Cathepsin H protein/polypeptide and can also be a means to detect specifically Cathepsin H protein/polypeptide harbouring one or more mutations regarding the size or the amino acid sequence in comparison to a wild type polypeptide/protein. A preferred examply of such a means is an antibody able to specifically detect Cathepsin H protein, e.g. for use in immunohistological or immunohistochemical techniques (e.g. detection of Cathepsin H protein or certain mutations thereof in histological tissue sections or Cathepsin H protein immobilized on suitable carriers like membranes, chips, ELISA plates etc.).

The means to detect Cathepsin H nucleic acid can e.g be a means to detect Cathepsin H mRNA /cDNA or genomic DNA, either wildtype or also harbouring one or more mutations regarding their length or their nucleic acid sequence in comparison to a wild type Cathepsin H nucleic acid. The means can e.g. be a means to specifically detect and/or quantify Cathepsin H mRNA and preferably comprises or is a specific Cathepsin H nucleic acid probe or a primer set capable of amplifying Cathepsin H DNA or, e.g. for use in PCR sequencing (for the detection of Mutations in the nucleotide sequence) or capable of amplifying Cathepsin H cDNA, e.g. for use in RT PCR (for the detection and/or quantification of Cathepsin H mRNA expression). Another means can e.g. be a nucleic acid probe able to specifically hybridise to Cathepsin H mRNA or cDNA under standard conditions, e.g. for use in Northern Blot or Chip hybridisation techniques.

The term wild type refers to the genotype or phenotype that is found in nature or in the standard laboratory stock for a given organism. According to one preferred embodiment, the wildtype sequences of Cathepsin H are the sequences according to SEQ ID NOs: 1, 2, 3, 4, 5, 6, 7, 8 and/or 9.

The design and synthesis of suitable primers is well known in the art (see also above). Primer sets for the detection of Cathepsin H could for example be the following:
Set 1: (Product length for detection of human CTSH transcript variant 1 = 154 nucleotides, product lengh for detection of human CTSH transcript variant 2=118 nucleotides):
   Forward primer 5'-GCGCTCCCAGTTGACGCTCT-3' (SEQ ID NO.10)
   Reverse primer 5'-CACGCACAGTTCGGCGGC-3' (SEQ ID NO.11)
Set 2: (Product length for detection of human CTSH transcript variant 1 = 153 nucleotides, product lengh for detection of human CTSH transcript variant 2=117 nucleotides):
   Forward primer 5'-CGCTCCCAGTTGACGCTCTGG-3' (SEQ ID NO.12)
   Reverse primer 5'-CACGCACAGTTCGGCGGC-3' (SEQ ID NO.13).

According to one embodiment of present invention, the means is a primer set for the amplification of Cathepsin H nucleic acid, and preferably a set of primers comprising at least one of the primers according to SEQ ID NOs. 10, 11 (together set 1), 12 and/or 13 (together set 2).

According to a further preferred embodiment of present invention, the means is a probe for the detection of Cathepsin H nucleic acid. The design and synthesis of suitable probes is well known in the art (see also standard literature below). According to yet another preferred embodiment of present invention, the means is an antibody for the specific detection of Cathepsin H protein or polypeptide.

The preparation of suitable antibodies or functional fragments thereof is well known in the art as well, e.g. by immunizing a mammal, for example a rabbit, with Cathepsin H protein or a fragment thereof, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well-known methods and, for example, purified by means of column chromatography. Monoclonal antibodies can, for example, be prepared in accordance with the known method of Winter & Milstein (Winter, G. & Milstein, C. (1991) Nature, 349, 293-299). Suitable procedures to produce Monoclonal antibodies are well known in the art as well (see e.g. literature for standard methods listed below). In the context of present invention, the term antibody or antibody fragment comprises also antibodies or antigen-binding parts thereof, which have been prepared recombinantly and, where appropriate, modified, such as chimaeric antibodies, humanized antibodies, multifunctional antibodies, bispecific or oligospecific antibodies, single-stranded antibodies and F(ab) or F(ab)₂ fragments (see, for example, EP-B1-0 368 684, US 4,816,567, US 4,816,397, WO 88/01649, WO 93/06213 or WO 98/24884). Cathepsin H antibodies are also commercially available, such as Goat Anti-Mouse Cathepsin H, Catalog# BAF1013, R&D Systems (Minneapolis, USA), Rat Anti-Mouse Cathepsin H, Catalog# MAB1013, R&D Systems (Minneapolis, USA), Goat Anti-Mouse Cathepsin H, Catalog# AF1013 or Rabbit Anti-Human Cathepsin H, Catalog# ABIN285430 (antibodies-online GmbH, Germany, see http://www.antikoerper-online.de) or Mouse Anti-Human Cathepsin H, Catalog#ABIN165388 (antibodies-online GmbH, Germany).

The production of Cathepsin H antibodies and the detection of human Cathepsin H from human tissue cytosols and sera is e.g. described in detail in Schweiger et al., Journal of Immunological Methods, 2001, p. 165-172(see e.g. p. 166-167 for the materials and methods).

Another aspect of present invention concerns a diagnostic kit for determining the pain and especially neuropathic pain sensitivity in an individual, which test kit comprises at least one means for the detection of Cathepsin H in a biological sample and its use.

In the context of the present invention, a test kit is understood to be any combination of the components identified in this application, which are combined, coexisting spatially, to a functional unit, and which can contain further components.

In the context of present invention, a test kit comprises at least a means for detection of Cathepsin H (e.g. amount/or mutation) in a biological sample, suitably together with suitable buffers and/or reagents for performing a detection reaction (e.g. immunological detection of Cathepsin H by means of an antibody, an enzymatic reaction for assaying Cathepsin H activity or the like). and/or sample preparation, and optionally a handling manual for performing the respective detection technique.

Other aspects of present invention concern methods of treatment, such as:
A method for treating pain in a subject that is experiencing pain comprising administering to said subject a therapeutically effective amount of a composition lowering the amount or activity of Cathepsin H in said subject. This can be the amount or activity of Cathepsin H altogether or in a certain tissue, e.g. in neural tissue, in lymphatic tissue or cells of the immune system such as mast cells, macrophages, neutrophils, T-cells (such as CD8+ T-cells), etc., wherein a therapeutically effective amount comprises an amount sufficient to ameliorate the pain sensation or sensitivity (especially with respect to neuropathic pain) in the individual.

A method for lowering the pain (and especially neuropathic pain) sensitivity in a subject comprising administering to said subject a therapeutically effective amount of a composition lowering the amount (e.g. expression, half life) or activity of Cathepsin H in said subject (e.g. in lymphatic or neural tissue or cells of the immune system), concerns yet another aspect of present invention.

Moreover, present invention concerns a method for modulating the pain (and especially neuropathic pain) sensitivity in an offspring from a non-human female subject comprising transferring (e.g. electroporating) a nucleic acid conferring a modulated Cathepsin H expression into Zygotes, transferring the Zygotes into a non-human foster mother and electing offspring according to its Cathepsin H expression characteristics (lowered or abolished Cathepsin H expression in comparison with wild type subjects, such as mice).

Another aspect of present invention concerns a compound that is able to lower Cathepsin H activity and/or expression for the treatment of pain and especially neuropathic pain.

Inhibitors of Cathepsin H are known in the art, such as E-64d (see e.g. Rüttger et al., BioTechniques, 41: 469-473, 2006) and Kirschke, H et al, 1995, Protein Profile 2: 1581-1643).

For the production of the medicament the modulators of Cathepsin H of the present invention can be formulated with suitable additives or auxiliary substances, such as physiological buffer solution, e.g. sodium chloride solution, demineralized water, stabilizers, such as protease or nuclease inhibitors, preferably aprotinin, ε-aminocaproic acid or pepstatin A or sequestering agents such as EDTA, gel formulations, such as white vaseline, low-viscosity paraffin and/or yellow wax, etc. depending on the kind of administration.

Suitable further additives are, for example, detergents, such as, for example, Triton X-100 or sodium deoxycholate, but also polyols, such as, for example, polyethylene glycol or glycerol, sugars, such as, for example, sucrose or glucose, zwitterionic compounds, such as, for example, amino acids such as glycine or in particular taurine or betaine and/or a protein, such as, for example, bovine or human serum albumin. Detergents, polyols and/or zwitterionic compounds are preferred.

The physiological buffer solution preferably has a pH of approx. 6.0-8.0, expecially a pH of approx. 6.8-7.8, in particular a pH of approx. 7.4, and/or an osmolarity of approx. 200-400 milliosmol/liter, preferably of approx. 290-310 milliosmol/liter. The pH of the medicament is in general adjusted using a suitable organic or inorganic buffer, such as, for example, preferably using a phosphate buffer, tris buffer (tris(hydroxymethyl)aminomethane), HEPES buffer

([4-(2-hydroxyethyl)piperazino]ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions.

The medicament can be administered in a conventional manner, e.g. by means of oral dosage forms, such as, for example, tablets or capsules, by means of the mucous membranes, for example the nose or the oral cavity, in the form of dispositories implanted under the skin, by means of injections, infusions or gels which contain the medicaments according to the invention. It is further possible to administer the medicament topically and locally in order to treat the particular joint disease as described above, if appropriate, in the form of liposome complexes. Furthermore, the treatment can be carried out by means of a transdermal therapeutic system (TTS), which makes possible a temporally controlled release of the medicaments. TTS are known for example, from EP 0 944 398 A1, EP 0 916 336 A1, EP 0 889 723 A1 or EP 0 852 493 A1.

Injection solutions are in general used if only relatively small amounts of a solution or suspension, for example about 1 to about 20 ml, are to be administered to the body. Infusion solutions are In general used if a larger amount of a solution or suspension, for example one or more litres, are to be administered. Since, in contrast to the infusion solution, only a few millilitres are administered in the case of injection solutions, small differences from the pH and from the osmotic pressure of the blood or the tissue fluid in the injection do not make themselves noticeable or only make themselves noticeable to an insignificant extent with respect to pain sensation. Dilution of the formulation according to the invention before use is therefore in general not necessary. In the case of the administration of relatively large amounts, however, the formulation according to the invention should be diluted briefly before administration to such an extent that an at least approximately isotonic solution is obtained. An example of an isotonic solution is a 0.9% strength sodium chloride solution. In the case of infusion, the dilution can be carried out, for example, using sterile water while the administration can be carried out, for example, via a so-called bypass.

According to one preferred embodiment of the different aspects of present invention, Cathepsin H, the derivative or fragment thereof can be used as an isolated molecule.

In the context of this invention, the term "isolated molecule", especially with respect to Cathepsin H, refers to Cathepsin H polynucleotides or polypeptides purified from natural sources as well as purified recombinant molecules (wherein the term purified comprises a partial purification as well as a complete purification).

The preparation of recombinant polypeptide or polynucleotide molecules and the purification of naturally occurring molecules from cells or tissue, as well as the preparation of cell- or tissue extracts is well known to the person of skill in the art (see e.g. also the standard literature listed below).

These comprise e.g. amplifying polynucleotides of desired length via the polymerase chain reaction (PCR) on the basis of the published genomic or coding polynucleotide sequences and the subsequent cloning of the produced polynucleotides in host cells (see e.g. standard literature listed below).

In the context of present invention, the term "polypeptide" refers to a molecule comprising amino acids bound to eachother by peptide bonds containing at least 50 amino acids coupled to eachother in a linear mode to form a polypeptide chain. Shorter molecules of this kind are referred to as peptides. The term "protein" refers to molecules comprising at least one polypeptide chain but can refer also to molecules comprising more than one polypeptide chains associated or bound to eachother. Thus, the term "protein" comprises the term "polypeptide" as well.

### Examples:

Materials and Methods:
Mouse strains used:
Five different inbred mouse strains were used: AKR/J (AKR), CBA/J (CBA), C3H/HeJ (C3H), C57BL/6J (B6) and C58/J (C58). Mice were obtained from The Jackson Laboratory (Bar Harbor, ME, USA). For these mice strains it has been shown that they differ significantly concerning several in vivo measures of pain (Mogil et al 1999)

Total RNA Isolation:
Total RNA from DRGs (dorsal root ganglia) was isolated with the PicoPure™ RNA Isolation Kit (Arcturus) following the manufacturer's instructions. RNA quality was assessed using the 2100 Bioanalyzer and RNA 6000 Nano LabChip™ kit (Agilent).

Affymetrix GeneChip™ Microarrays:
First-strand cDNA synthesis was performed using 500ng total RNA with a 100pM T7-(dT)24 oligomer (GGCCAGTGAATTGTAATACGACTCACTATAGGGAGGCGG-dT₂₄ SEQ ID NO:14) according to Baugh, L.R, Hill, A.A., Brown, E.L. and Hunter, C.P. (2001) Nucleic Acids Res. 29, e29 and SuperScript II Reverse Transcriptase following the manufacturers instructions. Double-stranded cDNA was synthesized and then extracted using phenol-chloroform followed by an ethanol precipitation step. An *in vitro* transcription reaction was performed with the doublestranded cDNA sample using the BioArray High Yield RNA Transcription Labeling kit (Enzo) according to the manufacturer's instructions. Transcription reactions were incubated at 37°C for 16h. cRNA was purified using the RNeasy™ Mini kit (Qiagen) protocol for RNA cleanup and quantified by a spectrophotometer. The biotin-labeled cRNA was fragmented using a RNA fragmentation buffer (200mM Tris-acetate, 500mM KOAc, 150mM MgOAc, pH 8.1), Hybridization and staining of Mouse Genome 430 2.0 GeneChips™ (Affymetrix) was performed according to the manufacturer's instructions. The microarrays were scanned using a GeneChip™ 3000 Scanner, and the scanned data were imported and analyzed using Resolver v5.1 expression data analysis software (Rosetta Biosoftware).

L5 spinal nerve transection and sham surgical procedures:
In anesthetized mice, the left L5 spinal nerve was exposed and the transverse process was then partially removed. After separation from the L4 spinal nerve, the L5 spinal nerve was transected. Sham surgery was identical to the L5 spinal nerve transection surgery, however, the L5 spinal nerve was not transected (see DeLeo et al. 2000)..

Determination of paw withdrawal threshold:
Paw withdrawal thresholds (PWTs) were assessed using a dynamic plantar aesthesiometer (see Szabo et al. 2005). After acclimation in a compartment with metal mesh floor, the stimulator was positioned under the animal's hindpaw, a straight metal filament driven by an electrodynamic actuator touched the plantar surface and exerted an increasing upward force until the animal removed the paw (paw withdrawal threshold, PWT) PWTs were assessed for hindpaws of the ipsilateral, operated side and of the contralateral side. Each animal was used at one occasion only. In all animal experiments the ethics guidelines for investigations in conscious animals were obeyed, and the procedures were approved by the local Ethics Committee

Correlational analysis:
For correlational analysis, the "pain phenotype" was defined for each nerve-transsected animal (Chung animal) as *C1 - S1,* where
*C1* = *In(ipsilateral PWT* / *contralateral PWT) and*
*S1* = *mean_{all sham animals within same strain} In(ipsilateral PWT* / *contralateral PWT).*
Two measures of differential transcriptional regulation were defined for each Chung animal and each measured gene based on its intensity expression data. The "raw intensity measure" was taken as the intensity measure computed by the Resolver expression data analysis software (v5.1) for the respective gene and animal. The "log ratio measure" was computed for a specific gene and Chung animal as *In(C2* / *S2),* where *C2 = Chung expression intensity* and *S2* = *mean_{all shem animals within same strain}*
*Sham expression intensity.*
Before correlations were computed, the set of genes was filtered to exclude genes that were expressed below noise level and without significant Chung vs. sham regulation. Eligible genes must be regulated in at least 60% of Chung animals with an absolute fold-change =>1.5 or in at least 20% of Chung animals with an absolute fold-change =>2.0. Also, corresponding gene expression had to be detectable ("present") in at least five animals as defined by a respective intensity p-value< 0.001. Pearson correlation coefficients for each gene between the pain phenotypic scores and one of the defined measures of transcriptional regulation were computed using the R software package (http://www.r-project.org/). Based on these, p-values of statistical significance and corresponding false-discovery rates (FDRs) were generated following the method of Storey et al. (2002). Genes with FDR< 0.05 under "log ratio measure" or "intensity measure" were considered significantly correlated.

Legend to the Figures:

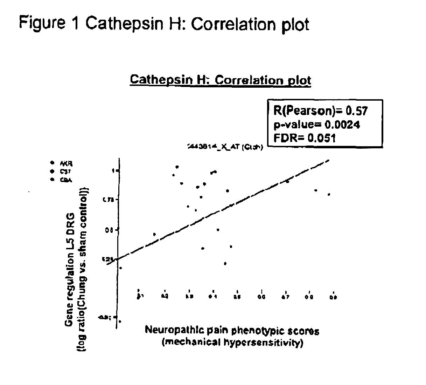

Figure 1: Cathepsin H - Correlation Plot
Figure 1 shows for every individual mouse its neuropathic pain phenotype (mechanical hypersensitivity, X-axis) and the corresponding gene regulation of Cathepsin H (log ratio(Chung vs. Sham control), Y-axis) in the L5 DRG. Mouse data are colour-coded depending on the used strain. A Pearson correlation analysis has been performed and revealed a significant positive correlation of the two parameters pain phenotype and Cathepsin H gene regulation. This means for individual mice that the higher the L5 DRG expression of Cathepsin H in Chung-operated neuropathic mice was, the more pronounced the mechanical hyperalgesia as exhibited in the behavioral test.

This significant correlation indicates a causal relationship of Cathepsin H gene expression for the induction of the neuropathic pain phenotype.

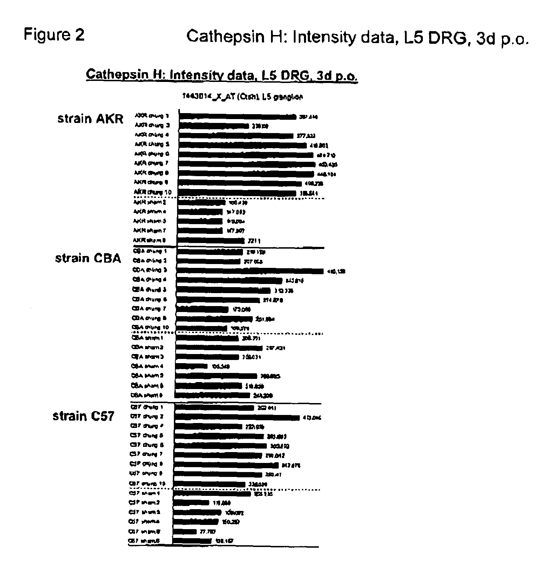

Figure 2: Cathepsin H - Intensity data Absolute values of Cathepsin H expression in L5 ganglia of the individual mice of the strains AKR, CBA and C57 after chung or sham surgery.

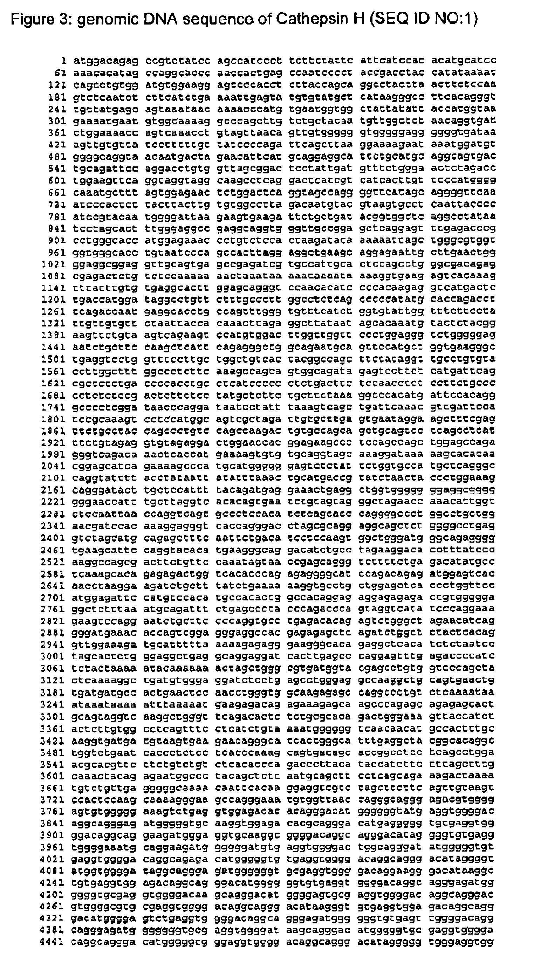

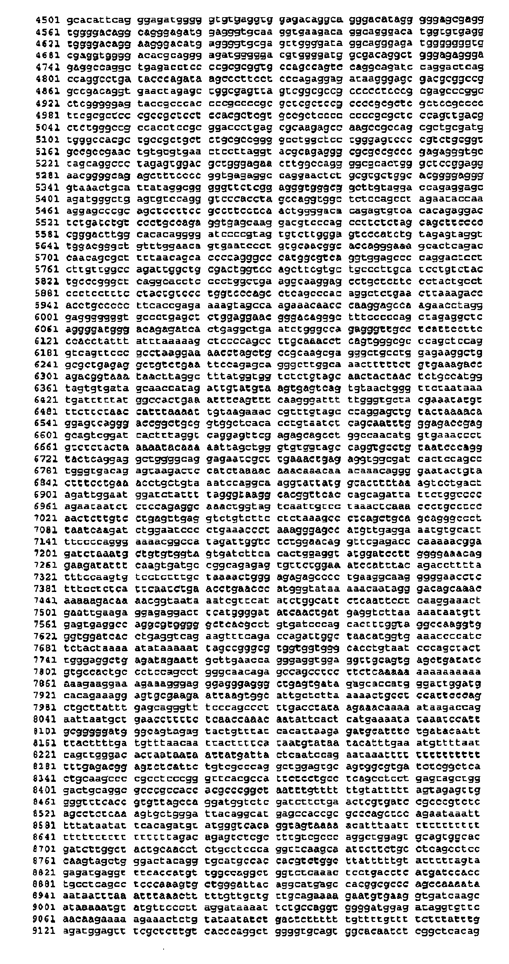

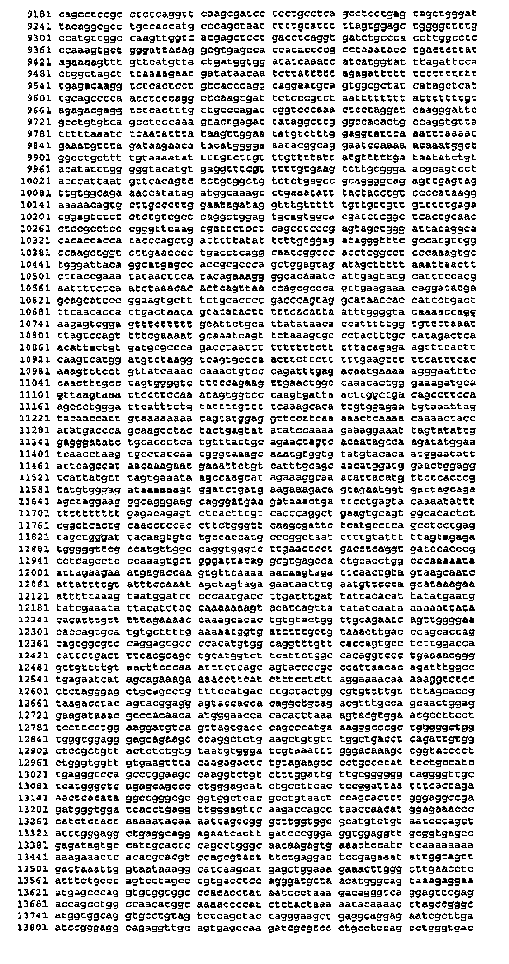

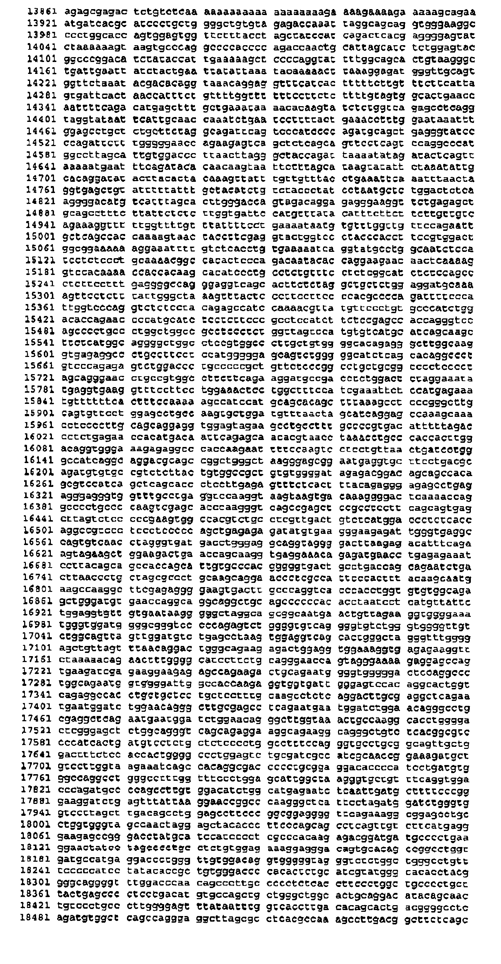

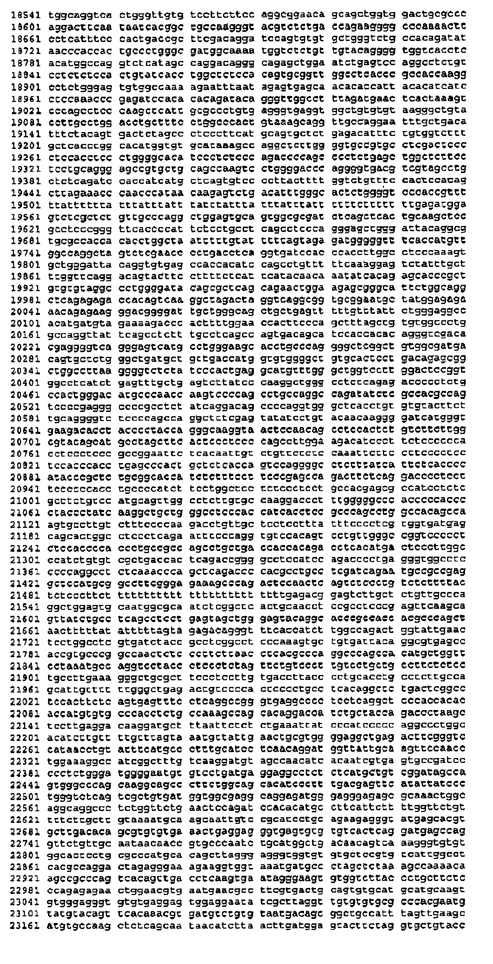

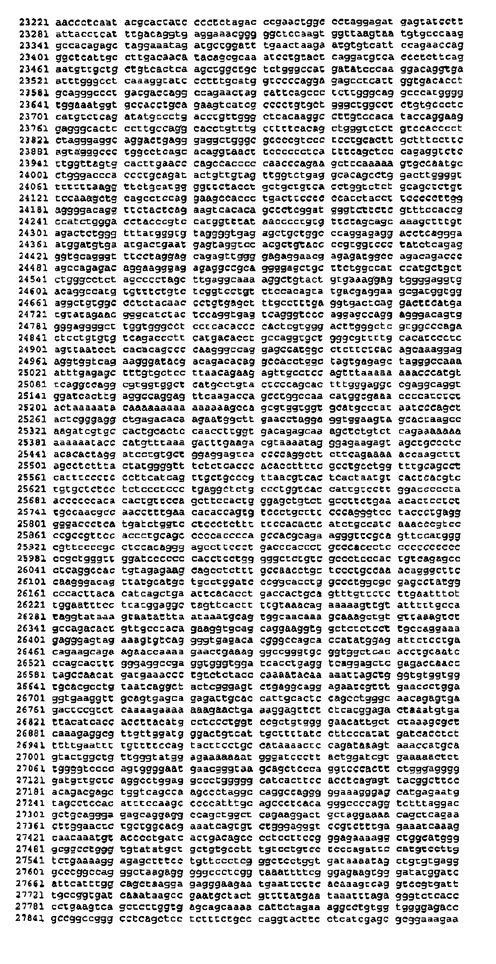

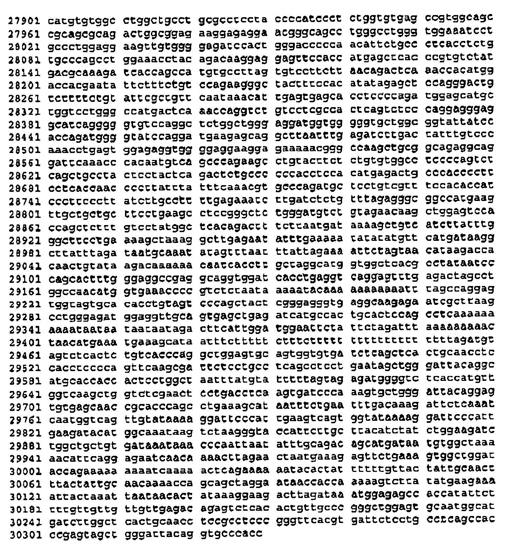

Figure 3: Genomic DNA sequence of homo sapiens Cathepsin H on chromosome 15 according to NCBI Reference Sequence: NG_009614.1 (SEQ ID NO. 1).

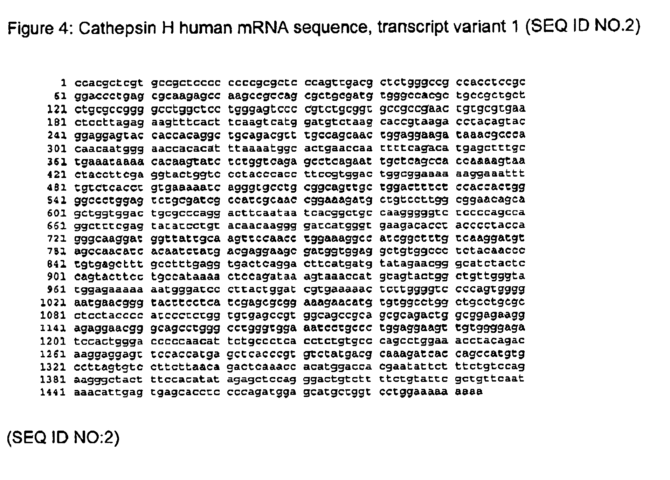

Figure 4: Coding sequence of homo sapiens Cathepsin H transcript variant 1 (SEQ ID NO:2) according to NM_004390.3 having a length of 1494 bp and encoding the longer isoform A of Cathepsin H.

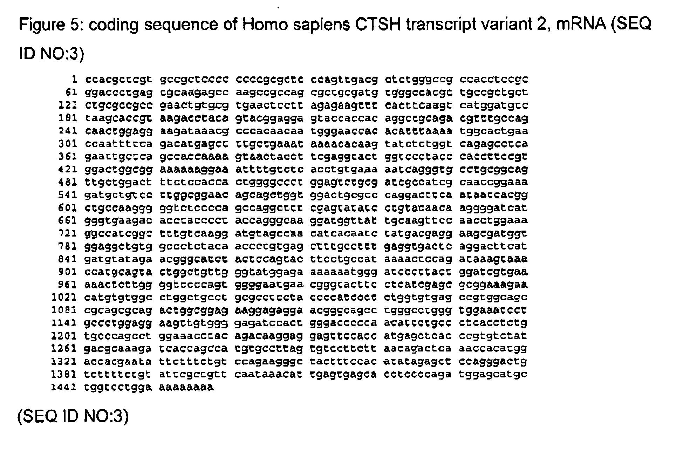

Figure 5: Coding sequence of homo sapiens Cathepsin H transcript variant 2 (SEQ ID NO:3) according to NM_148979.2 having a length of 1458 bp and encoding the shorter isoform B of Cathepsin H. According to the above NCBI entry, this transcript variant lacks an alternative in-frame segment compared to variant 1 resulting in a shorter protein (isoform B) when compared to isoform A encoded by transcript variant 1. This may result in a protein (isoform B) that may more likely be a secreted than a lysosomal protein

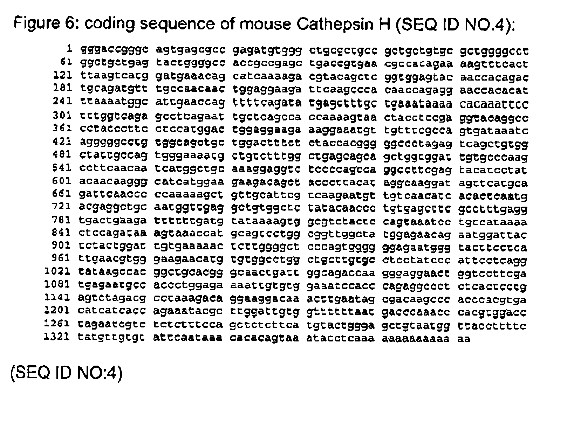

Figure 6: coding sequence of mus musculus Cathepsin H (SEQ ID NO.4) according to NCBI entry BC006878.1.
Figure 7: human Cathepsin H protein sequence according to UniProtKB/Swiss-Prot P09668 (SEQ ID NO.5) comprising 335 amino acids and constituting the preproform of Human Cathepsin H (isoform A). This sequence is further processed into a mature form; it is cleaved into the following 3 chains: Cathepsin H mini chain Cathepsin H heavy chain, Cathepsin H light chain (light and heavy chain together may be referred to as large chain); all chains are held together by disulfide bonds. Amino Acids 1-22 constitute the signal peptide (22 aa long), amino acids 23-97 constitute the activation peptide (75 aa long), aminoacids 98-105 constitute the Cathepsin H mini chain (8 aa long), amino acids 106-115 constitute the propeptide (10 aa long), amino acids 116-335 constitute the Cathepsin H long chain (220 aa long) consisting of the heavy and light chain held together by disulfide bonds, amino acids 116-292 constitute the Cathepsin H heavy chain (177 aa long), amino acids 293-335 constitute the Cathepsin H light chain (43 aa long).

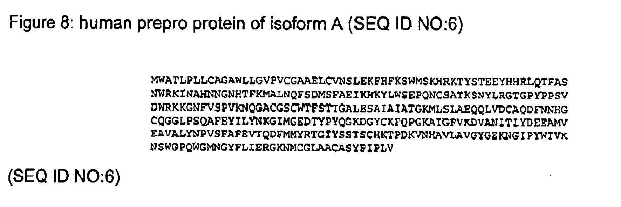

Figure 8: Human isoform A prepro protein according to NM_004390.3 (SEQ ID NO:6; translated amino acid sequence of SEQ ID NO.2).

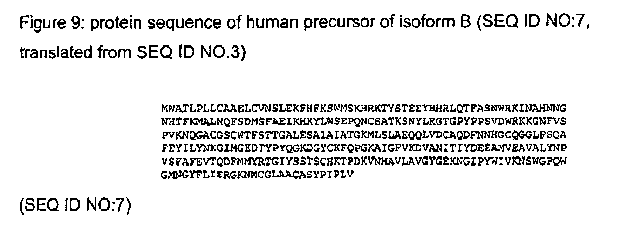

Figure 9: Human isoform B prepro protein according to NM_148979.2 (SEQ ID NO:7; translated amino acid sequence of SEQ ID NO:3).

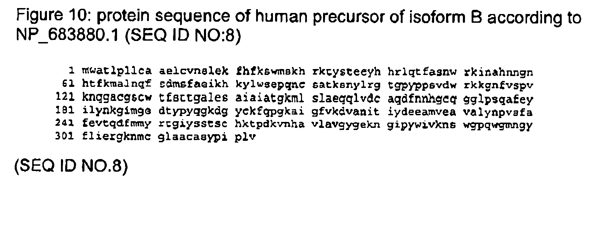

Figure 10: protein sequence of human Cathepsin H isoform a preproprotein according to NP_004381.2 (SEQ ID NO.8).

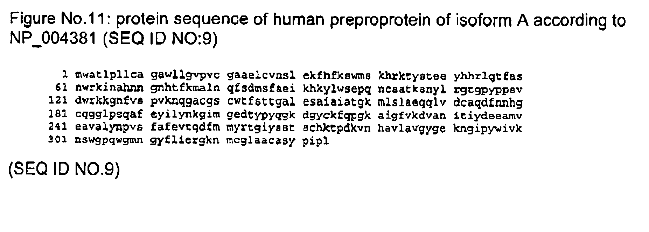

Figure 11: protein sequence of human Cathepsin H isoform b precursor protein according to NP_683880.1 (SEQ ID NO.9).

### References:

DeLeo JA et al (2000) Transgenic expression of TNF by astrocytes increases mechanical allodynia in a mouse neuropathy model. Neuroreport 11:599-602.
Storey JD. (2002) A direct approach to false discovery rates. Journal of the Royal Statistical Society, Series B, 64: 479-498.
Szabo A et al. (2005) Role of transient receptor potential vanilloid 1 receptors in adjuvant-induced chronic arthritis: in vivo study using gene-deficient mice. J. Pharmacol. Exp. Ther. 314:111-119.
Julius and Basbaum " Molecular mechanisms of nociception", Nature, volume 413, 13. September 2001, pp. 203 - 209;
Scholz and Woolf "Can we conquer pain" , Nature neuroscience supplement, volume 5, November 2002, pp. 1062 - 1067;
Wood, J.D. "Pathobiology of Visceral Pain: Molecular Mechanisms and Therapeutic Implications II. genetic approaches to pain therapy", American Journal pf Physiological Gastrointestinal Liver Physiology, 2000, volume 278, G507-G512;
Woolf and Mannion "Neuropathic pain: aetiology, symptoms mechanisms, and management", The LANCET, volume 353, June 5, 1999, pp. 1959 - 1964;
Woolf J. and Salter M.W. "Neuronal Plasticity: Increasing the Gain in Pain", Science, volume 288, June 9, 2000, pp. 1765-1768;
Pham, C. T. N.; Armstrong, R. J.; Zimonjic, D. B.; Popescu, N. C.; Payan, D. G.; Ley, T. J. "Molecular cloning, chromosomal localization, and expression of murine dipeptidyl peptidase I" J. Biol. Chem. 272: 10695-10703, 1997.
Pham, C. T. N.; Ley, T. J. :"Dipeptidyl peptidase I is required for the processing and activation of granzymes A and B in vivo". Proc. Nat. Acad. Sci. 96: 8627-8632, 1999. Rao, N. V.; Rao, G. V.; Hoidal, J. R. :"Human dipeptidyl-peptidase I". J. Biol. Chem. 272: 10260-10265, 1997.
Manour, S., Thomas, K.R., and Capecchi, M.R., 1989, " disruption of the proto-oncogene Int-2 in mouse embryo-derived stem cells: a general strategy for targeting mutations so non-selectable genes", Nature 336, 348-352.
Soriano, PI, Montgomery, C., Geske, R., and Bradley, A., 1991, "Targeted disruption of the c-src proto-oncogene leads to osteopetrosis in mice", Cell 65, 693-702.
Turk, B., Turk, D., and Turk, V., Lysosomal cysteine proteases: more than scavengers. Biochim Biophys Acta. 2000 Mar 7; 1477(1-2):98-111.
Turk B., Turk V. and Turk D., 1997, Structural and Functional Aspects of Papain-Like Cysteine Proteinases and Their Protein Inhibitors, Biological Chemistry, Vol. 378, pp. 141-150;
Turk V., Turk. B and Turk. D, 2001, Lysosomal Cysteine Proteases: Facts and Opportunities, The EMBO Journal, Vol. 20, No.17 pp. 4629-4633.
Rüttger, A, Mollenhauer, J., Löser, R., Gütschow, M., and Wiederanders, B.,Microplate assay for quantitative determmination of cathepsin activities in viable cells using derivatives of 4-methoxy-β-naphthylamide, BioTechniques 41: 469-473 (October 2006);
Kirschke, H., A.J. Barrett, and N.D. Rawlings, 1995, Proteinases 1: lysosomal cysteine proteinases: Protein Profile 2: 1581-1643).
Ishidoh K., Suzuki, K., Katunuma, N., and Kominami, E., Gene Structures of Rat Cathepsins H and L., Biomedica and Biochimica Acta, 1991; 50 (4-6): 541-7.
Ishidoh K., Kominami EI, Katunuma N. and Suzuki K., 1989, Gene structure of rat cathepsin H, FEBS Letters Volume 253, number 1,2, 103-107;
Rothe M, and Dodt J, 1992, Studies on the Aminopeptidase Activity of Rat Cathepsin H, European Journal of Biochemistry, 210, 759-764
Vasiljeva O., Dolinar M., Turk V and Turk B., 2003, Recombinant Human Cathepsin H Lacking the Mini Chain is an Endopeptidase, Biochemistry 2003, 42, 13522-13528;
Schweiger A, Stabuc B., Popovic T, Turk V and Kos J., 1997, Enzyme-linked immunosorbent assay fort he detection of total cathepsin H in human tissue cytosols and sera, Journal of Immunological Methods 201 (1997) 165-172;
Koga, H., Mori N., Yamada H., Nishimura Y, Kazuo T., Kato K. and Imoto T., 1991, Rat Cathepsin H-Catalyzed Transacylation: Comparisons of the Mechanism and the Specificity with Papain-Superfamily Proteases, Journal of Biochemistry 110, 939-944.

### Literature for standard laboratory methods

If not indicated otherwise, standard laboratory methods were or can be performed according to the following standard literature:
Sambrook et al. (1989) Molecular Cloning: A Laboratory Manual. Second edition. Cold Spring Harbor Laboratory Press, Cold Spring Harbor, NY. 545 pp;
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly uptdated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated; Wiley & Sons, Inc; Editors: John E. Coligan, Ben M. Dunn, Hidde L. Ploegh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994;
Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York"
Transgenic Animal Technology A Laboratory Handboook. C.A, Pinkert, editor; Academic Press Inc., San Diego, California, 1994 (ISBN: 0125571658)
Gene targeting: A Practical Approach, 2nd Ed., Joyner AL, ed. 2000. IRL Press at Oxford University Press, New York;
Manipulating the Mouse Embryo: A Laboratory Manual. Nagy, A, Gertsenstein, M., Vintersten, K., Behringer, R., 2003, Cold Spring Harbor Press, New York;
Remington's Pharmaceutical Sciences, 17th Edition, 1985 (for physiologically tolerable salts (anorganic or organic), see esp. p. 1418)

Standard Literature for Laboratory Methods:
If not indicated otherwise, laboratory methods were or can be performed according to standard methods listed in the below standard literature:
   Sambrook et al. (1989) Molecular Cloning: A Laboratory Manual. Second edition. Cold Spring Harbor Laboratory Press, Cold Spring Harbor, NY. 545 pp or Current Protocols in Molecular Biology;
Current Protocols in Molecular Biology; regularly updated, e.g. Volume 2000; John Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly uptdated, e.g. Volume 2003; John Wiley & Sons, Inc; Editors: Nicholas C. Dracopoli, Honathan L. Haines, Bruce R, Korf, Cynthia C. Morton, Christine E. Seidman, J.G. Seigman, Douglas R. Smith.
Current Protocols in Protein Science; regularly updated, e.g. Volume 2003; John Wiley & Sons, Inc; Editors: John E. Coligan, Ben M. Dunn, Hidde L. Ploegh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994;
Gene Targeting: a practical approach (1995), Editor: A.L. Joyner, IRL Press
Remington's Pharmaceutical Sciences, Edition 17, 1985.

## Claims

1. The use of Cathepsin H for identifying compounds that modulate neuropathic pain

2. The use of a non-human transgenic animal heterologously expressing Cathepsin H for identifying or analyzing compounds that modulate neuropathic pain.

3. The use of a non-human transgenic animal heterologously expressing Cathepsin H as a model system for enhanced neuropathic pain sensitivity.

4. The use of a non-human Cathepsin H knock-out animal for identifying or analyzing compounds that modulate neuropathic pain.

5. The use of a non-human Cathepsin H knock-out animal as a model system for lowered neuropathic pain sensitivity.

6. The use of a cell heterologously expressing Cathepsin H or a functional fragment thereof for identifying compounds that modulate neuropathic pain.

7. The use of a cell heterologously expressing Cathepsin H or a functional fragment thereof as a model system for enhanced neuropathic pain sensitivity.

8. The use of a Cathepsin knock-out cell for identifying or analyzing compounds that modulate neuropathic pain.

9. The use of a Cathepsin knock-out cell as a model system for lowered neuropathic pain sensitivity.

10. The use of a cell heterologously expressing a reporter gene expressibly linked to the Cathepsin H promoter and/or enhancer or a functional fragment thereof for identifying or analyzing compounds that modulate neuropathic pain.

11. A method of identifying or analyzing compounds modulating and/or preventing neuropathic pain, comprising the steps
f. Providing at least two samples;
g. Contacting one sample containing Cathepsin H or a functional fragment or derivative thereof with a compound,
h. determining the activity of Cathepsin H in the presence of compound,
i. determining the activity of Cathepsin H in the absence of compound, and
j. comparing the activity of Cathepsin H according to c) with that according to d).

12. A method for identifying or analyzing compounds that modulate and/or prevent neuropathic pain comprising:
c. Contacting a Cathepsin H protein or functional fragment or derivative thereof with a test compound; and
d. Determining whether the test compound modulates the activity of the Cathepsin H protein or functional fragment or derivative thereof.

13. A Method for identifying or analyzing compounds that modulate and/or prevent neuropathic pain comprising:
a. Contacting a cell, which has a detectable amount or activity of Cathepsin H or of a functional fragment or derivative thereof, with a test compound;
b. Determining whether the test compound is able to modulate the amount or activity of Cathepsin H or the functional fragment or derivative thereof present in the cell.

14. A method for identifying or analyzing compounds that modulate and/or prevent neuropathic pain comprising:
a. Contacting a nucleic acid coding for a Cathepsin H protein or a functional fragment or derivative thereof with a test compound in a transcriptionally active system, and
b. Determining the amount of mRNA coding for the Cathepsin H protein or the functional fragment or derivative thereof present in said system in presence of said compound, and
c. Determining whether the compound is capable of modulating the amount of mRNA coding for the Cathepsin H protein or functional fragment or derivative present in said system.

15. A method for identifying compounds or analyzing compounds that modulate and/or prevent neuropathic pain comprising:
a. Providing a cell transfected with a nucleic acid vector comprising the promoter of a Cathepsin H gene or a functional fragment thereof operationally coupled to a reporter gene or a functional fragment thereof:
b. Providing a cell transfected with a control vector which comprises a reporter gene or a functional fragment thereof not being operationally coupled do a functional Cathepsin H promoter;
c. Determining the reporter gene activity of the cell according to a) and b) in the presence of a test compound;
d. Determining the reporter gene activity of the cell according to a) and b) in absence of the test compound.

16. A method for identifying or analyzing a compound that modulates neuropathic pain comprising
c. Selecting a compound that modulates the activity of Cathepsin H as a test compound, and
d. Administering said test compound to a subject in sensation of pain to determine whether the pain is modulated.

17. A method of identifying or analyzing a compound that modulates and/or prevents neuropathic pain in a subject comprising:
a. Assaying a biological activity of Cathepsin H or a functional fragment or derivative thereof in the presence of one or more test compounds to identify one or more modulating compounds that modulate the biological activity of Cathepsin H, and
b. Testing one or more of the modulating compounds for their ability to reduce pain, pain sensation or pain sensitivity in a subject.


SEQUENCE LISTING

<110>  Sanofi-Aventis

<120>  Use of Cathepsin H

<130>  DE2009/140

<150>  09290659.3
<151>  2009-08-31

<160>  14

<170>  PatentIn version 3.3

<210>  1
<211>  30329
<212>  DNA
<213>  Homo sapiens

<400>  1
atggacagag ccgtctatcc agccatccct tcttctattc attcatccac acatgcatcc      60

aaacacatag ccaggcaccc aaccactgag ccaatcccct accgacctac catataaaat     120

cagcctgtgg atgtggaagg agtcccacct cttaccagca ggcctactta acttctccaa     180

gtctcaatct cttcatctga aaattgagta tgtgtatgct cataagggcc ttcacagggt     240

tgttatgagc agtaaataac aaaacccatg tgaatggtgg ctattatatt accatggtaa     300

gaaaatgaat gtggcaaaag gcccagcttg tctgctacaa tgttggctct aacaggtgat     360

ctggaaaacc agtcaaacct gtagttaaca gttgtggggg gtgggggagg ggggtgataa     420

agttgtgtta tcctttttgt tatccccaga ttcagcttaa ggaaaagaat aaatggatgt     480

ggggcaggta acaatgacta gaacattcat gcaggaggca ttctgcatgc aggcagtgac     540

tgcagattcc aggacctgtg gttagcggac tcctattgat gtttctggga actctagacc     600

tggaagttca ggtaggtagg caagcctcag gactcatcgt catcacttgt tcccatgggg     660

caaatgcttt agtggagaac tctggactca ggtagccagg ggttcatagc aggggttcaa     720

atcccactct tacttacttg tgtggcctta gacaatgtac gtaagtgcct caattacccc     780

atccgtacaa tggggattaa gaagtgaaga ttctgctgat acggtggctc aggcctataa     840

tcctagcact ttgggaggcc gaggcaggtg ggttgccgga gctcaggagt ttgagacccg     900

cctgggcacc atggagaaac cctgtctcca ctaagataca aaaaattagc tgggcgtggt     960

ggtgggcacc tgtaatccca gccacttagg aggctgaagc aggagaattg cttgaactgg    1020

ggaggcggag gttgcagtga gccgagatcg tgccattgca ctccagcctg ggcgacagag    1080

cgagactctg tctccaaaaa aactaaataa aaataaaata aaaggtgaag agtcacaaag    1140

tttatttgtg tgaggcactt ggagcagggt ccaacacatc cccacaagag gtcatgactc    1200

tgaccatgga taggcctgtt ctttgccctt ggcctctcag cccccatatg caccagacct    1260

tcagaccaat gaggcacctg ccagtttggg tgtttcatct ggtgtattgg tttcttccta    1320

ttgttgtgtt ctaattacca caaacttaga ggcttataat agcacaaatg tactctacgg    1380

aagttctgta agtcagaagt ccatgtggac ttggttggtt ccctggaggg tctgggggag    1440

aatctgcttc caagctcatt cagagggctg gcagaatgca gttccatgct ggtgaagggc 1500
tgaggtcctg gtttccttgc tggctgtcac tacggccagc ttctataggt tgcctgtgta 1560
ccttggcttt ggccctcttc aaagccagca gtggcagata gagtccttct catgattcag 1620
cgctctctga ccccacctgc ctcatccccc ctctgacttc tccaacctct ccttctgccc 1680
cctctctccg actcttctcc tatgctcttc tgcttctaaa ggcccacatg attccacagg 1740
gcccctcgga taacccagga taatcctatt taaagtcagc tgattcaaac gttgattcca 1800
tccgcaaagt cctccatggc agtcgctaga ttgtgcttga gtgaatagga agctttcgag 1860
ttctgcctac cagccctgtc cagccaagac tgtgccagca gctgcagtcc tcagcctcat 1920
ttctgtagag gtgtagagga ctggaaccac ggagaagccc tccagccagc tggagccaga 1980
gggtcagaca aactcaccat gaaaagtgtg tgcaggtagc aaaggataaa aagcacacaa 2040
cggagcatca gaaaagccca tgcatggggg gagtctctat tctggtgcca tgctcagggc 2100
caggtatttt acctataatt atatttaaac tgcatgaccg tatctaacta ccctggaaag 2160
cagggatact tgctccattt tacagatgag gaaactgagg ctggtggggg ggaggcgggg 2220
gggaaccatt tgcttaggtc acacagtgaa tctgtagtag ggctagaacc aaacattggt 2280
ctccaattaa ccaggtcagt gccctccaca tctcagcacc caggggccct ggcctgctgg 2340
aacgatccac aaaggagggt caccagggac ctagcgcagg aggcagctct ggggcctgag 2400
gtctagcatg cagagctttc aattctgaca tcctccaagt ggctgggatg ggcagagggg 2460
tgaagcattc caggtacaca tgaagggcag gacatctgcc tagaaggaca cctttatccc 2520
aaggccagcg acttctgttc caaatagtaa ccgagcaggg tcttttctga gacatatgcc 2580
tcaaagcaca gagagactgg tcacacccag agaggggcat ccagacagag atggagtcac 2640
aacctaagga agatctgctt tatctgaaaa aaggtgcctg ctggagctca ccctggttcc 2700
atggagattc catgtccaca tgccacactg gccacaggag aggagagaga ctgtggggga 2760
ggctctttaa atgcagattt ctgagcccta cccagaccca gtaggtcata tcccaggaaa 2820
gaagtccagg aatctgcttc cccaggtgcc tgagacacag agtctgggct agaacatcag 2880
gggatgaaac accagtcgga gggaggccac gagagagctc agatctggct ctactcacag 2940
gttggaaaga tgcattttta aaaagagagg gaagggcaca gaggctcaca tctctaatcc 3000
tagcactctg ggaggctgag gcaggaggat cacttgagcc caggagtttg agaccccatc 3060
tctactaaaa atacaaaaaa actagctggg cgtgatggta cgagcctgtg gtcccagcta 3120
ctcaaaaggc tgatgtggga ggatctcctg agcctgggag gccaaggctg cagtgaactg 3180
tgatgatgcc actgaactcc aacctgggtg gcaagagagc caggccctgt ctcaaaataa 3240
ataaataaaa atttaaaaat gaagagacag agaaagagca agcccagagc agagagcact 3300
gcagtaggtc aaggctgggt tcagacactc tctgcgcaca gactgggaaa gttaccatct 3360
actcttgtgg cctcagtttc ctcatctgta aaatgggggg tcaacaacat gccactttgc 3420
aaggtgatga tgtaagtgaa gaacaggcca tcactgggca tttgaggcta cggcacaggc 3480

tggtctgaat caccctctcc tcacccaaag cagtgacagc accggcctcc tcagcctgga 3540
acgcacgttc ttctgtctgt ctcacaccca gacccttaca taccatcttc tttagctttg 3600
caaactacag agaatggccc tacagctctt aatgtagctt cctcagcaga aagactaaaa 3660
tgtctgttga gggggcaaaa caattcacaa ggaggtcgtc tagcttcttc agttgtaagt 3720
ccactccaag caaaagggaa gccagggaaa tgtggttaac cagggcaggg agacgtgggg 3780
agtgtggggg aaagtctgag gtggagacac acagggacat ggggggtatg aggtggggac 3840
aggcagggag atgggggtgc aaggtggaga cacgcaggga catgaggggg tgtgaggtgg 3900
ggacaggcag gaagatggga ggtgcaaggc ggggacaggc agggacatag gggtgtgagg 3960
tggggaaatg caggaagatg gggggatgtg aggtggggac tggcagggat atgggggtgt 4020
gaggtgggga caggcagaga catgggggtg tgaggtgggg acaggcaggg acataggggt 4080
atggtgggga taggcaggga gatggggggt gcgaggtggg gacaggaagg gacataaggc 4140
tgtgaggtgg agacaggcag ggacatgggg ggtgtgaggt ggggacaggc agggagatgg 4200
ggggtgcgag gtggggacaa gcagggacat ggggagtgcg aggtggggac aggcagggac 4260
gtggggcgtg cgaggtgggg acaggcaggg acataagggt gtgaggtgga gacaggcagg 4320
gacatgggga gtctgaggtg gggacaggca gggagatggg gggtgtgagt tggggacagg 4380
cagggagatg ggggggtgcg aggtggggat aagcagggac atgggggtgc gaggtgggga 4440
caggcaggga catgggggtg ggaggtgggg acaggcaggg acataggggg tgggaggtgg 4500
gcacattcag ggagatgggg gtgtgaggtg gagacaggca gggacatagg gggagcgagg 4560
tggggacagg cagggagatg gagggtgcaa ggtgaagaca ggcagggaca tggtgtgagg 4620
tggggacagg aagggacatg aggggtgcga gttggggata ggcagggaga tgggggggtg 4680
cgaggtgggg acacgcaggg agatggggga cgtggggatg gcgacaggct gggagaggga 4740
gaggccaggc tgagacctcc ccgcgcggtg ccagccagtc caggcagatc caggactcag 4800
ccaggcctga tacccagata agcccttcct cccagaggag ataagggagc gacgcggccg 4860
gccgacaggt gaactagagc tggcgagtta gtcggcgccg ccccctcccg cgagcccggc 4920
ctcgggggag taccgcccac cccgccccgc gctcgctccg ccccgcgctc gctccgcccc 4980
ttcgcgctcc cgccgctcct ccacgctcgt gccgctcccc ccccgcgctc ccagttgacg 5040
ctctgggccg ccacctccgc ggaccctgag cgcaagagcc aagccgccag cgctgcgatg 5100
tgggccacgc tgccgctgct ctgcgccggg gcctggctcc tgggagtccc cgtctgcggt 5160
gccgccgaac tgtgcgtgaa ctccttaggt acgcagaggg cgcgccgccc gagagggtgc 5220
tagcaggccc tagagtggac gctgggagaa cttggccagg ggcgcactgg gctccggagg 5280
aacggggcag agctttcccc ggtgagaggc caggaactct gcgtgctggc acggggaggg 5340
gtaaactgca ttataggcgg gggttctcgg agggtgggcg gcttgtagga ccagaggagc 5400
agatgggctg agtgtccagg gtcccaccta gccaggtggc tctccagcct agaataccaa 5460
aggagcccgc agctccttcc gccttcctca actggggaca cagagtgtca cacagaggac 5520

tctgatctgt ccctgccaga ggtgagcaag gacgtcccag ccttctctag cagcttcccc 5580
cgggacttgg cacacagggg atccccgtag tgtcttggga gtcccatctg tagagtaggt 5640
tggacgggct gtttggaaca gtgaatccct gtgcaacggc accagggaaa gcactcagac 5700
caacagcgct tttaacagca ccccagggcc catggcgtca ggtggagccc caggactcct 5760
cttgttggcc agattggctg cgactggtcc agcttcgtgc tgcccttgca tcctgtctac 5820
tgcccgggct caggcacctc ccctggctga aggcaaggag cctgctcttc cctactgcct 5880
ccctcctttc ctactgtccc tggtcccagc ctcagcccac aggctctgaa cttaaagacc 5940
acctgccccc ttcaccgaga aaagtagcca agaaacaacc caaggagcca agaacctagg 6000
gagggggggt gccctgagct ctggaggaac gggacagggc tttcccccag ctagaggctc 6060
aggggatggg acagagatca ctgaggctga atctgggcca gagggttgcc tcattccttc 6120
ccacctattt atttaaaaag ctccccagcc ttgcaaacct cagtgggcgc ccagctccag 6180
gtcagttccc gcctaaggaa aacctagctg ccgcaagcga gggctgcctg gagaaggctg 6240
gcgctgagag gctgtctgaa ttccagagca gggcttggca aactttttct gtgaaagacc 6300
agacggtaaa taacttaggc tttatggtgg tctctgtagc aactactaac tctgccatgg 6360
tagtgtgata gcaaccatag attgtatgta agtgagtcag tgtaactggg ttctaataaa 6420
tgatttttat ggccactgaa atttcagttt caagggattt ttgggtgcta cgaaatatgt 6480
ttcttctaac catttaaaat tgtaagaaac cgtttgtagc ccaggagctg tactaaaaca 6540
ggagtcaggg accggctgcg gtggctcaca cctgtaatct cagcaatttg ggagaccgag 6600
gcagtcggat cactttaggt caggagttcg agagcagcct ggccaacatg gtgaaaccct 6660
gtctctacta aaaatacaaa aattagctgg gtgtggtagc caggtgcctg taatcccagg 6720
tactcaggag gctggggcag gagaatcgct tgaaactgag aggtggcgat cactccagcc 6780
tgggtgacag agtaagactc catctaaaac aaacaaacaa acaaacaggg gaatactgta 6840
ctttcctgaa acctgctgta aatccaggca aggtattatg gcacttttaa agtcctgact 6900
agattggaat ggatctattt tagggtaagg cacggttcac cagcagatta ttctggcccc 6960
agaataatct ctccagaggc aaactggtag tcaattgtcc taaactcaaa ccctgcctcc 7020
aactcttgtc ctgagttgag gtctgtcttc ctctaaagcc ctcagctgca gcagggccct 7080
taatcaagat ctggaatccc ctgaaaccct aaagggagcc atgttgagga aatgtgcatt 7140
ttccccaggg aaaacggcca tagattggtc tctggaacag gttcgagacc caaaaacgga 7200
gatctaaatg ctgtgtggta gtgatcttca cactggaggt atggatcctt ggggaaacag 7260
gaagatattt caagtgatgc cggcagagag tgttctggaa atccatttac agacctttta 7320
tttccaagtg tcctctttgc taaaactggg agagagcccc tgaaggcaag ggggaacctc 7380
tttcctctca ttcaatctga acctgaaccc atgggtataa aaacaatagg gacagcaaac 7440
aaaaagacaa aacggtaata aatcgttcat atctggcatt ctcaattcct caaggaaact 7500
gaattgaaga ggagaggact tcatggggat atcaactgat gaggtcttaa aaataatgtt 7560

gagtgaggcc aggcgtgggg gctcacgcct gtgatcccag cactttggta ggccaaggtg 7620
ggtggatcac ctgaggtcag aagtttcaga ccagattggc taacatggtg aaaccccatc 7680
tctactaaaa atataaaaat tagccgggcg tggtggtggg cacctgtaat cccagctact 7740
tgggaggctg agatagaatt gcttgaacca gggaggtgga ggttgcagtg agctgatatc 7800
gtgccactgc cctccagcct gggcaacaga gccagcctcc ttctcaaaaa aaaaaaaaaa 7860
aaagaaggaa agaaagggag ggagggaggg ctgagtgata gagcaccatg ggattggatg 7920
cacagaaagg agtgcgaaga attaagtggc attgctctta aaaactgcct ccattcccag 7980
ctgcttattt gagcagggtt tcccagccct ttgacctata agaaacaaaa ataagaccag 8040
aattaatgct gaaccttttc tcaaccaaac aatattcact catgaaaata taaatccatt 8100
gcgggggatg ggcagtagag tactgtttat cacattaaga gatgcatttc tgatacaatt 8160
ttacttttga tgtttaacaa ttacttttca taatgtataa tacatttgaa atgttttaat 8220
cagttgggac actaataata attatgatta ctcaatccag aataaatttt tttttttttt 8280
tttgagacgg agtctcattc tgtcgcccag gctggagtgc agtggcgtga tctcggctca 8340
ctgcaagccc cgcctcccgg gttcacgcca ttctcctgcc tcagcctcct gagtagctgg 8400
gactgcaggc gcccgccacc acgcccggct aatttgtttt ttgtattttt agtagagttg 8460
gggtttcacc gtgttagcca ggatggtctc gatcttctga actcgtgatc cgcccgtctc 8520
agcctctcaa agtgctggga ttacaggcat gagccaccgc gcccagctcc agaataaatt 8580
tttataatat tcacagatgt atgggtcaca ggtagtaaaa acatttaatt tttttttttt 8640
tttttttttt ttttttagac agagtctcgc tttgtcgccc aggctggagt gcagtggcac 8700
gatcttggct actgcaacct ctgcctccca ggttcaagca attcttctgc ctcagcctcc 8760
caagtagctg ggactacagg tgcatgccac cacgtctggc ttatttttgt atttttagta 8820
gagatgaggt ttcaccatgt tggccaggct ggtctcaaac tcctgacctc atgatccacc 8880
tgcctcagcc tcccaaagtg ctgggattac aggcatgagc cacggcgccc agccaaaata 8940
aataatttaa atttaaactt tttgttgttg ttgcagaaaa gaatgtgaag gtgatcaagc 9000
ataaaaatgt atgttccctt aggataaaat tctgccaggt ggggatggag ataggtgttc 9060
aacaagaaaa agaaactctg tataatatct gactcttttt tgttttgttt tctttatttg 9120
agatggagtt tcgctcttgt cacccaggct ggggtgcagt ggcacaatct cggctcacag 9180
cagcctccgc ctctcaggtt caagcgatcc tcctgcctca gcctcctgag tagctgggat 9240
tacaggcgcc tgccaccatg cccagctaat tttgtatttt ttagtggagc tggggttttg 9300
ccatgttggc caagttggtc atgagctcct gacctcaggt gatctgccca ccttggcctc 9360
ccaaagtgct gggattacag gcgtgagcca ccacaccccg cctaaatacc tgactcttat 9420
agaaaagttt gttcatgtta ctgatggtgg atatcaaatc atcatggtat ttagattcca 9480
ctggctagct ttaaaagaat gatataacaa tcttattttt agagattttt tttttttttt 9540
tgagacaagg tctcactcct gtcacccagg caggaatgca gtggcgctat catagctcat 9600

tgcagcctca acctcccagg ctcaagtgat tctcccgtct aatttttttt atttttttgt 9660
agagacgagg tctcactttg ttgcccagac tggtcccaaa ctcctaggct caagggattc 9720
gcctgtgtca gcctcccaaa gtactgagat tataggcttg ggccacactg ccaggtgtta 9780
tttttaaatc tcaatattta taagttggaa tatgtctttg gaggtattca aatttaaaat 9840
gaaatgttta gataagaaca tacatgggga aatacggcag gaatccaaaa acaaatggct 9900
ggcctgcttt tgtaaaatat tttgtcttgt ttgttttatt atgtttttga taatatctgt 9960
acatatttgg gggtacatgt gaggtttcgt ttttgtgaag tcttgcggga acgcagtcct 10020
acccattaat gttcacagtc tctgtggctg tctctgagcc gcaggggcag agttgagtag 10080
ttgtggcaga aaccatatag atggcaaagc ctgaaatatt tactacctgt ccccataagg 10140
aaaaacagtg cttgcccttg gaatagatag gtttgttttt tgttgttgtt gtttttgaga 10200
cggagtctct ctctgtcgcc caggctggag tgcagtggca cgatctcggc tcactgcaac 10260
ctccgcctcc cgggttcaag cgattctcct cagcctcccg agtagctggg attacaggca 10320
cacaccacca tacccagctg atttttatat ttttgtggag acagggtttc gccatgttgg 10380
ccaagctggt cttgaacccc tgacctcagg caatcggccc acctcggcct cccaaagtgc 10440
tgggattaca ggcatgagcc accgcgccca gctggagtag atagcttttt aaattaactt 10500
cttaccgaaa tataacttca tacagaaagg ggcacaaatc attgagtatg catttccacg 10560
aattttctca atctaaacac actcagttaa ccagcgccca gttgaagaaa caggatatga 10620
gcagcatccc ggaagtgctt tctgcacccc gacccagtag gcataaccac catcctgact 10680
ttcaacacca ttgactaata gtatatactt tttcacatta atttggggta caaaaccagg 10740
aagagtcgga gtttcttttt gcattctgca ttatataaca ccatttttgg tgttttaaat 10800
ttagtccagt tttcgaaaat gcaaatcagt tctaaagtgc cctactttgc tatagactca 10860
acattactgt gatgcgccca gacctaattt tttttttctt tttacagaga agtttcactt 10920
caagtcatgg atgtctaagg tcagtgccca acttcttctt tttgaagttt ttcatttcac 10980
aaagtttcct gttatcaaac caaactgtcc cagatttgag acaatgaaaa agggaatttc 11040
caactttgcc tagtggggtc tttccagaag ttgaactggc caaacactgg gaaagatgca 11100
gttaagtaaa ttccttccaa atagtggtcc caagtgatta acttggctga cagccttcca 11160
agccctggga ttcatttctg tattttgttt tcaaagcaca ttgtggagaa tgtaaattag 11220
tacaaccatt gtaaaaaaaa cagtatggag gttccatcaa aaactcaaaa caaaactacc 11280
atatgaccca gcaagcctac tactgagtat atatccaaaa gaaaggaaat tagtatattg 11340
gagggatatc tgcaccctca tgtttattgc agaactagtc acaatagcca agatatggaa 11400
tcaacctaag tgcctatcaa tgggtaaagc aaatgtggtg tatgtacaca atggaatatt 11460
attcagccat aacaaagaat gaaattctgt catttgcagc aacatggatg gaactggagg 11520
tcattatgtt tagtgaaata agccaagcat agaaaggcaa atattacatg ttctcactcg 11580
tatgtgggag ataaaaaagt ggatctgatg aagaaagaca gtagaatggt gactagcaga 11640

agctaggaag ggcagggaag cagggatgaa gataaactga ttcctgagta caaaatattt 11700
tttttttttt gagacagagt ctcactttgt cacccaggct gaagtgcagt ggcacactct 11760
cggctcactg caacctccac cttctgggtt caagcgattc tcatgcctca gcctcctgag 11820
tagctgggat tacaagtgtc tgccaccatg cccggctaat ttttgtattt ttagtagaga 11880
tgggggttcg ccatgttggc caggtgggtc ttgaactcct gacctcaggt gatccacccg 11940
cctcagcctc ccaaagtgct gggattacag gcgtgagcca ctgcacctgg cccaaaaata 12000
attagaagaa atgagaccaa gtgttcaaaa aacaagtaga ttcaactgta gtaagcaatc 12060
attattttgt atttccaaat agctagtaga gaataatttg aatgttccca gcataaagaa 12120
atttttaaag taatggatct cccaatgacc ttgatttgat tattacacat tatatgaatg 12180
tatcgaaata ttacatttac caaaaaaagt acatcagtta tatatcaata aaaaattata 12240
cacatttgtt tttagaaaac caaagcacac tgtgtactgg ttgcagaatc agttggggaa 12300
caccagtgca tgtgcttttg aaaaatggtg atctttgctg taaacttgac ccagcaccag 12360
cagtggcgtc caggagtgcc ccacatgtgg caggtttgtt caccagtgcc tcttggacca 12420
cattctgact ttcacgcagc tgcatggtct tcattctggc cacaggtccc tgaaaacggg 12480
gttgttttgt aacttcccaa atttctcagc agtaccccgc ccattaacac agatttggcc 12540
tgagaatcat agcagaaaga aaaccttcat ctttcctctt aggaaaacaa aaaggtctcc 12600
ctctagggag ctgcagcctg tttccatgac ttgctactgg cgtgttttgt tttagcaccg 12660
taagacctac agtacggagg agtaccacca caggctgcag acgtttgcca gcaactggag 12720
gaagataaac gcccacaaca atgggaacca cacatttaaa agtacgtgga acgccttcct 12780
tccttcctgg aaggatgtca gttagtgacc cagcccatga aagggcccgc tgggggctgg 12840
tgggtggagg gagcagaagc ccaggctctg aagctgtgtt tggctgacct cagattgtgg 12900
ctccgctgtt actctctgtg taatgtggga tcgtaaattt gggacaaagc cggtacccct 12960
ctgggtggtt gtgaagttta caagagactc tgtagaagcc cctgccccat tcctgccatc 13020
tgagggtcca gcctggaagc caaggtctgt ctttggattg ttgcgggggg taggggttgc 13080
tcatgggctc agagcagccc ctgggagcat ctgccttcac tccggattaa tttcactaga 13140
aactcacata ggccgggcgc ggtggctcac gcctgtaatt ccagcacttt gggaggccga 13200
gatgggtgga tcacctgagg ttgggagttc aagaccagcc taaccaacat ggagaaaccc 13260
catctctact aaaaatacaa aattagccgg gcttggtggc gcatgtctgt aatcccagct 13320
atttgggagg ctgaggcagg agaatcactt gatcccggga ggtggaggtt gcggtgagcc 13380
gagatagtgc cattgcactc cagcctgggc aacaagagtg aaactccatc tcaaaaaaaa 13440
aaagaaactc acacgcacgt ccagcgtatt ttctgaggac tctgagaaat attggtagtt 13500
gactaaattg gtaataaagg catcaagcat gagctggaaa gaaacttggg cttgaacctc 13560
atttctgccc agtcctagcc tgtgacctcc agggatgcta acatgggcag taaagaggaa 13620
atgagcccag gtgtggtggc ccacacctat aatccctaaa gacagggtca ggagttcgag 13680

accagcctgg ccaacatggc aaaaccccat ctctactaaa aatacaaaac ttagccgggc 13740
atggtggcag gtgcctgtag tctcagctac tagggaagct gaggcaggag aatcgcttga 13800
atccgggagg cagaggttgc agtgagccaa gatcgcgtcc ctgcctccag cctgggtgac 13860
agagcgagac tctgtctcaa aaaaaaaaaa aaaaaaaaga aaagaaaaga aaaagcagaa 13920
atgatcacgc atccctgctg gggctgtgta gagaccaaat taggcagcag gtgggaaggc 13980
ccctggcacc agtggagtgg ttctttacct agctatccat cagactcacg aggggagtat 14040
ctaaaaaagt aagtgcccag gccccacccc agaccaactg cattagcatc tctggagtac 14100
ggcccggaca tctataccat tgaaaaagct ccccaggtat tttggcagca ctgtaagggc 14160
tgattgaatt atctactgaa ttatattaaa tacaaaaact taaaggagat gggttgcagt 14220
ggttctaaat acgacacagg taaacaggag gtttcatcac tttttcttgt ttcttcatta 14280
gtgattcact aaccatttct gttttggttt tttccttctc ttttgtagtg gcactgaacc 14340
aattttcaga catgagcttt gctgaaataa aacacaagta tctctggtca gagcctcagg 14400
taggtataat tcattgcaac caaatctgaa tccttttact gaaacctttg gaataaattt 14460
ggagcctgct ctgcttctag gcagattcag tcccatcccc agatgcagct gagggtatcc 14520
ccagattctt tgggggaacc agaagagtca gctctcagca gttcctcagt ccaggcccat 14580
ggccttagca ttgtggaccc ttaacttagg gctaccagat taaaatatag atactcagtt 14640
aaaaatgaat ttcagataca caacaagtaa ttctttagca taagtatatt ctaaatattg 14700
cacaggatat acttacacta caaagttatt tgttgtttac ctgaaattca aatttaacta 14760
ggtgagctgt atttttattt gctacatctg tctaccctat cctaatgctc tggactctca 14820
aggggacatg tcatttagca cttgggacca gtagacagga gagggaaggt tctgagagct 14880
gcagcctttc ttattctctc ttggtgattc catgtttata catttcttct tcttgttgtc 14940
agaaaggttt ttggttttgt ttattttcct gaaaataatg tgtttggttg ttccagaatt 15000
gctcagccac caaaagtaac taccttcgag gtactggtcc ctacccacct tccgtggact 15060
ggcggaaaaa aggaaatttt gtctcacctg tgaaaaatca ggtatgcctg gcaatctcca 15120
tcctctccct gcaaaacggc cacactccca gacaatacac caggaagaac aactcaaaag 15180
gtccacaaaa ccaccacaag cacatccctg cctctgtttc ctctcggcat ctctccagcc 15240
ctcttccttt gaggggccag ggaggtcagc acttctctag gctgctctgg aggatgcaaa 15300
agttcctctt tattgggcta aagtttactc ccttccttcc ccacgcccca gatttcccca 15360
ttggtcccag gtctctccta cagagccatc caaaacgtta tgttccctgt gcccatctgg 15420
acaccagaac cccatgcatc tcctcttccc gcctccatct tctccgagcc accagggtcc 15480
agcccctgcc ctggctggcc gcctccctct ggttagtcca tgtgtcatgc atcagcaagc 15540
ttctcatggc aggggctggc ctccgtggcc cttgctgtgg ggcacagagg gcttggcaag 15600
gtgagaggcc ctgccttcct ccatggggga gcagtctggg ggcatctcag cacaggccct 15660
gtcccagaga gtctggaccc tgcccccgct gttctcccgg cctgctgcgg cccctcccct 15720

agcagggaac ctgccgtggc cttcttcaga aggatgccga cctctggact ctaggaaata 15780
tgaggtgaag gtttccttcc tggaaactcc tggctttcca tcgaaattct ccatgagaaa 15840
tgttttttca ctttccaaaa agccatccat gcagcacagc tttaaagcct cccgggcttg 15900
cagtgttcct ggagcctgcc aagtgctgga tgtttaacta gcatcaggag ccaaagcaaa 15960
cctccccttg cagcaggagg tggagtagaa gcctgccttt gccccgtgac atttttagac 16020
cctctgagaa ccacatgaca attcagagca acacgtaacc taaacctgcc caccacttgg 16080
acaggtggga aagagaggcc caccaagaat tttccaagtc ctcctgttaa ctgatcctgg 16140
gccatcaggc aggacgcagc cggctgggct aagggagcgg aatgaggtgc ttcctgacgc 16200
agatgtgtgc cgtctcttac tgtggccgct gtgtggggat agagacggac agcagccaca 16260
gcgtccatca gctcagcacc ctccttgaga gtttctcact ttacagaggg agagcctgag 16320
agggagggtg gtttgcctga ggtccaaggt aagtaagtga caaaggggac tcaaaaccag 16380
gcccctgccc caagtcgagc acccaagggt cagccgagct ccgcctcctt cagcagtgag 16440
cttagtctcc cccgaagtgg ccacgtctgc ctcgttgact gtctcatgga ccctctcacc 16500
aggccgtccc tccctccccc agctgagaga gatatgtgaa gggaagagat tgggtgaggc 16560
cagtgtcaac ctagggtgat gacctgggag gcaggtaggg gacttaagag acatttcaga 16620
agtagaagct ggaagactga accagcaagg tgaggaaaca gagatgaacc tgagagaaat 16680
ccttacagca gccaccagca ttgtgcccac gggggtgact gcctgaccag cagaatctga 16740
cttaaccctg ctagcgccct gcaagcagga accctcgcca ttcccacttt acaagcaatg 16800
aagccaaggc ttcgagaggg gaagtgactt gcccaggtca cccacctggt gtgtggcaga 16860
gctgggatgt gaaccaggca ggcaggctgc agccccccac acctaatcct catgttattc 16920
tggaggtgtt gtgaataagg gggctaggca gcggcaatga actgttagaa ggtggggaaa 16980
tgggtggatg gggcgggtcc cccagagtct ggggtgtcag gggtgtctgg gtggggttgt 17040
ctggcagtta gttggatgtc tgagcctaag tggaggtcag cactgggcta gggtttgggg 17100
agctgttagt ttaacaggac tgggcagaag agactggagg tggaaaggtg agagaaggtt 17160
ctaaaaacag aactttgggg catccttctg cagggaacca gtagggaaaa gaggagccag 17220
tgaagatcga gaaggaagag agccagaaga ctgcagaatg gggtggggga ctccaggccc 17280
tggcagaatg gtggggattg gccaccaaga ggtggtgatt gggagtccac aggcactggt 17340
cagaggccac ctgctgctcc tgctcctttg caagcctctc aggacttgcg aggctcagaa 17400
tgaatggatc tggaacaggg cttgcgagcc tcagaatgaa tggatctgga acagggcctg 17460
cgaggctcag aatgaatgga tctggaacag ggcttggtaa actgccaagg cacctgggga 17520
ctcgggagct ctggcagggt cagcagagga aggcagaagg cagggctgtc tcacggcgtc 17580
cccatcactg atgtcctctg ctctcccctg gcctttccag ggtgcctgcg gcagttgctg 17640
gactttctcc accactgggg ccctggagtc tgcgatcgcc atcgcaaccg gaaagatgct 17700
gtccttggta agaaatcagc cacaggcgac cccctgcgga ggacacccca tcctgatgtg 17760

ggccaggcct gggcctttgg tttccctgga gcattggcta agggtgctgt ttcaggtgga 17820
cccagatgcc ccagccttgt ggacatctgg catgagaatc tcaattgatg cttttcccgg 17880
gaaggatctg agtttattaa ggaaccggcc caagggctca ttcctagatg gatctgggtg 17940
gtccttagct tgacagcctg gagccttccc ggcggagggg ttcagaaagg cggagcctgc 18000
ctggtgggta gccaactagg agctacaccc ttcccagcag cctcagttgt cttcatgagg 18060
gaagagccgg gacctatgca tccatcccct cgcccacaag agacggatga tgcccctgaa 18120
ggaactatcc tagcccctgc ctctgtggag aaaggaggga cagtgcacag ccggcctggt 18180
gatgccatga ggaccctggg ttgtggacag gtgggggtag ggtctctggc tgggcctgtt 18240
tcccccatcc tatacaccgc tgtgggaccc cacactctgc atcgtatggg cacacctacg 18300
gggcaggggt ttggacccaa cagcccttgc cccctctcac cttccctggc tgcccctgct 18360
tactgagccc ctcccgacat gtgccagctg ctgggctggc actgcaggac atacagcaac 18420
tgtccctgcc cttggggagt ttataatttg gtcaccttga cacagcactg acggggcctc 18480
agatgtggct cagccaggga ggcttagcgc ctcacgccaa agccttgacg gcttctcagc 18540
tggcaggtca ctgggttgtg tccttcttcc aggcggaaca gcagctggtg gactgcgccc 18600
aggacttcaa taatcacggc tgccaagggt acgtctctga ccagaagggg cccaaaactt 18660
cctcatttcc cactgaccgc ttcgacagga tccagtgtgt gctgggtctg ccacagatat 18720
aacccaccac tgccctgggc gatggcaaaa tggtctctgt tgtacagggg tggtcacctc 18780
acatggccag gtctcatagc caggacaggg cagagctgga atctgagtcc aggcctctgt 18840
cctctctcca ctgtatcacc tggctctcca cagtgcggtt ggcctcaccc gccaccaagg 18900
cctctgggag tgtggccaaa agaatttaat agagtgagca acacaccatt acacatcatc 18960
ctccaaaccc gagatccaca cacagataca gggttggcct ttagatgaac tcactaaagt 19020
cccagcctcc caagctcatt gcgccctgtg agggtgaggt ggctgtgtgt aagggctgta 19080
ccttgcctgg acctgctttc ctggcccact gtaaagcagg ttgccaggaa tttgctgaca 19140
tttctacagt gactctagcc ctcccttcat gcagtgctct gagacatttc tgtggtcttt 19200
gctcacctgg cacatggtgt gcataaagcc aggctcttgg ggtgccgtgc ctcgactccc 19260
ctccacctcc ctggggcaca tccctctccc agaccccagc ccctctgagc tggctcttcc 19320
tcctgcaggg agccgtgctg cagccaagtc ctggggaccc aggggtgacg tcgtagcctg 19380
ctctcagatc caccatcatg ctcagtgtcc ccttactttt ggtctgtttc cactccacag 19440
cttagaaacc caacccataa caagagtctg acatttgggc actctggggt cccaccgttt 19500
ttatttttta tttatttatt tatttattta tttatttatt ttttcttttt ttgagatgga 19560
gtctcgctct gttgcccagg ctggagtgca gtggcgcgat ctcagctcac tgcaagctcc 19620
gcctcccggg ttcaccccat tctcctgcct cagcctccca gggagctggg attacaggcg 19680
tgcgccacca cacctggcta atttttgtat ttttagtaga gatggggttt tcaccatgtt 19740
ggccaggcta gtctcgaacc cctgacctca ggtgatccac ccaccttggc ctcccaaagt 19800

gctgggatta caggtgtgag ccaccacatc cagcctgttt ttcaaaggag tctatttgct 19860
ttggttcagg acagtacttc cttttctcat tcatacaaca aatatcacag agcacccgct 19920
gtgtgtaggc cctggggata cagcgctcag cagaactgga agagcgggca ttctggcagg 19980
ctcagagaga ccacagtcaa ggctagacta ggtcaggcgg tgcggaatgc tatggagaga 20040
aacagagaag ggacggggat tgctgggcag ctgctgagtt tttgtttatt ctgggaggcc 20100
acatgatgta gaaaagaccc acttttggaa ccacttccca gctttagctg tgtggccctg 20160
gccaggttat tcagcctctt tgcctcagcc agtgacagca tccaccacac agggtcgaca 20220
cgaggggtca gggagtcatg cctgggaagc acctgcccag gggctcggct gtggcgatga 20280
cagtgctctg ggctgatgct gttgaccatg gtgtggggct gtgcactcct gacagagcgg 20340
ctggccttaa ggggtctcta tcccactgag gcatgtttgg gctggtcctt ggactccggt 20400
ggcctcatct gagtttgctg agtcttatcc caaggctggg cctctcagag accccctctg 20460
ccactgggac atgcccaacc aagtccccag cctgccaggc cagatatctc gccacgccag 20520
tccccgaggg ccccgcctct atcaggacag ccccaggtgg gctcacctgt gtgtacttct 20580
tgcaggggtc tccccagcca ggctttcgag tatatcctgt acaacaaggg gatcatgggt 20640
gaagacacct acccctacca gggcaaggta actccaacag cctccactct gtcttcttgg 20700
cgtacagcat gcctagcttc actccctccc cagccttgga agacatccct tctcccccca 20760
cctccctccc gccggaattc tcacaattgt ctgttctctc caaattcttc cttcccctcc 20820
tccacccacc tgagcccact gctctcacca gtccaggggc ctctttatca ttctcacccc 20880
atacccgctc tgcggcacca tctctttcct tcccgagcca gacttctcag gacccctcct 20940
tccccccacc tgccccatct tcctggctcc tctccctcct gccacgagcg ccatcctctc 21000
gcctctgtcc atgcagttgg cctcttgtgc caaggaccct ttgggggccc acccccaccc 21060
ctaccctatc aaggctgctg ggcctcccac catcacctcc gcccagcctg gccacagcca 21120
agtgccttgt ctttccccaa gacctgttgc tcctccttta tttcccctcg tggtgatgag 21180
cagcactggc ctccctcaga atttcccagg tgtccacagt cctgttgggc cggtccccct 21240
ctccacccca ccctgccgcc agcctgctga ccaccacaga cctcacatga ctcccttggc 21300
ccatctgtgt cgctgaccac tcagaccggg gcctccatcc agacccctga gggtggcctc 21360
ccccaggcct ctcaaaccca gctcagaccc cacgcctgcc tcgatcagaa tgccgccgag 21420
gctccatgcg gccttcggga gaaagcccag actccaactc agtctccctg tctcttttac 21480
tctcccttct tttttttttt tttttttttt ttttgagacg gagtcttgct ctgttgccca 21540
ggctggagtg caatggcgca atctcggctc actgcaacct ccgcctcccg agttcaagca 21600
gttatctgcc tcagcctcct gagtagctgg gagtacaggc acccgccacc acgcccagct 21660
aactttttat atttttagta gagacagggt ttcaccatct tggccagact ggtattgaac 21720
tcctggcctc gtgatctacc gcctcggcct cccaaagtgc tgtgattaca ggcgtgagcc 21780
accgtgcccg gccaactctc ccttcttaac ctcacgccca ggcccagcca catgctggtt 21840

cctaaatgcc aggtcctacc ctccctctag ttctgtccct tgtcctgctg ccttctctcc 21900
tgccttgaaa gggctgcgct tccctccttg tgaccttacc cctgcacctg cctcttgcca 21960
gcattgtttt ttgggctgag actgtcccca ctcccctgcc tcacaggctc tgactcggcc 22020
tccacttctc agtgagtttc ctcaggccgg gtgaggcccc tcctcaggct cccaccacac 22080
accatgtgtg cccacctctg ccaaagccag cacaggacca tctgctacca gaccctaagc 22140
tccttgagga caaggatgct ttaattctct ctgaaattat cccatccccc aggccctggc 22200
acatcttgtt ttgtttagta aatgttattg aactgcgtgg ggaggctgag acttcgggtc 22260
cataacctgt atttcatgcc ctttgcatcc tcaacaggat ggttattgca agttccaacc 22320
tggaaaggcc atcggctttg tcaaggatgt agccaacatc acaatcgtga gtgccgatcc 22380
ccctctggga tggggaatgt gtcctgatga ggaggcctct ctcatgctgt cggatagcca 22440
gtgggcccag caaggcagcc cttctggcag cacatccctt tgacgagttc atattatccc 22500
tgggtctcag tcgctgtgat ggtggcgagg caggagatgg gagggagagc gcaaactggc 22560
aggcaggccc tctggttctg aactccagat ccacacatgc cttcattctt ttggttctgt 22620
tttcttgctt gtaaaatgca agcaattgtc cgcatcctgc agaagagggt atgagcacgt 22680
gcttgacaca gcgtgtgtga aactgaggag ggtgagtgtg tgtcactcag gatgagccag 22740
gttctgttgc aataacaacc gtgcccaatc tgcatggctg acaacagtca aagggtgtgt 22800
ggcactcctg cgcccatgca cagcttaggg agggtggtgt gtgctccgtg tcattggcct 22860
cacgccagga ctagagggaa agaaggtggt aaatgatgcc ctagctctaa agccaaaaca 22920
agccgcccag tcacagttga cctcaagtga atagggaagt gtggtcttac cctgcttctc 22980
ccagagagaa ctggaacgtg aatgaacgcc ttcgtgactg cagtgtgcat gcatgcaagt 23040
gtgggagggt gtgtgaggag tggaggaata tcgcttaggt tgtgtgtgcg cccacgaatg 23100
tatgtacagt tcacaaacgt gatgtctgtg taatgacagc ggctgccatt tagttgaagc 23160
atgtgccaag ctctcagcaa taacatctta acttgatgga gtacttctag gtgctgtacc 23220
aaccctcaat acgcactatc ccctctagac ccgaactggc cctaggagat gagtatcctt 23280
attacctcat ttgacaggtg aggaaacggg ggctccaagt ggttaagtaa tgtgcccaag 23340
gccacagagc taggaaatag atgctggatt tgaactaaga atgtgtcatt ccagaaccag 23400
ggctcattgc cttgacaaca tacagcgcaa atcctgtact caggatgtca ccctcttcag 23460
aatgttgctg ctgtcactca agctggctgc tctgggccat gatatcccaa ggacaggtga 23520
atttgggcct caaaggtatc cctttgcatg gtccccagga gagcctcatt ggtgacacct 23580
gcagggccct gacgaccagg ccagaactag cattcagccc tcttgggcag gcccatgggg 23640
tggaaatggt gccacctgca gaagtcatcg cccctgtgct gggctggcct ctgtgccctc 23700
catgtctcag atatgccctg acctgttggg ctcacaaggc cttgtccaca taccaggaag 23760
gagggcactc ccttgccagg cacctgtttg cttttcacag ctgggtctct gtccacccct 23820
ctagggaggc aggactgagg gaggctgggc gccccgtccc tcctgcactt gctttccttc 23880

agtagggccc tggcctcagc acaggtaact tcccccctca tttcagctcc cagaggtctc 23940
ttggttagtg cacttgaacc cagccacccc caacccagaa gctccaaaaa gtgccaatgc 24000
ctgggaccca ccctgcagat actgttgtag ttggtctgag gcacagcctg gacttggggt 24060
ttttttaagg ttctgcatgg ggttctacct gctgctgtca cctggtctct gcagctctgt 24120
tccaaagctg cagcctccag gaagccaccc tgacttcccc ccacctacct tcccccttgg 24180
aggggacagg ttctactcag aagtcacaca gccctcggat gggtcttctc gtttccaccg 24240
ccatctggga cctacccgtc catggtttat aacccctgtg ttctagcagc aaagctttgt 24300
agactctggg tttatgggtg taggggtgag agctgctggc ccaggagagg acctcaggga 24360
atggatgtga atgactgaat gagtaggtcc acgctgtacc ccgtggtccc tatctcagag 24420
ggtgcagggt ttcctaggag cagagttggg gagaggaacg agagatggcc agacagaccc 24480
agccagagac aggaagggag agaggccgca ggggagctgc ttctggccat ccatgctgct 24540
ctgggcctct agcccctagc ttgaggcaaa aggctgtact gtgaaaggag tggggaggtg 24600
acaggccatg tgtttctgtc tcggtcctgt ttccacagta tgacgaggaa gcgatggtgg 24660
aggctgtggc cctctacaac cctgtgagct ttgcctttga ggtgactcag gacttcatga 24720
tgtatagaac gggcatctac tccaggtgag tcagggtccc aggagccagg agggacagtg 24780
gggaggggct tggtgggcct cctcacaccc cactcgtggg acttgggctc gtggcccaga 24840
ctcctgtgtg tcagaccctt catgacacct gccaggtgct gggcgttttg cacatccctc 24900
agttaatcct cacacagccc caagggccag gagccatggc ctcttctcac agcaaaggag 24960
aggtggtcag aagggatacg acagacacag gccacctggc tagtgagagc tagggccaaa 25020
atttgagagc tttgtgctcc ttaacagaag agttgcctcc agtttaaaaa aaacccatgt 25080
tcaggccagg cgtggtggct catgcctgta ctcccagcac tttgggaggc cgaggcaggt 25140
ggatcacttg aggccaggag ttcaagacca gcctggccaa catggcgaaa ccccatctct 25200
actaaaaata caaaaaaaaa aaaaaagcca gcgtggtggt gcatgcctat aatcccagct 25260
actcgggagg ctgagacaca agaatggctt gaacctagga ggtggaagta gcactaagcc 25320
aagatcgtgc cactgcactc caaccttggt gacagagcaa agctctgtct cagaaaaaaa 25380
aaaaaatacc catgtttaaa gatttgaaga cgtaaaatag ggagaagagt agctgccctc 25440
acacactagg atcctgtgct ggaggagtca ccccaggctt cttcagaaaa accaagcttt 25500
agcctcttta ctatggggtt tctctcaccc acaccttttc gcctgcctgg tttgcagcct 25560
catttccctc ccttcatcag ttgctgcccg ttaacgtcac tcactaatgt cactcacgtc 25620
tgtgctctcc tctccctccc tgaggctctg ccctggtcac cattgtcctt ggacccccta 25680
accccccaca cactgttcca gcttccactg ggagctgtct gccttctgaa acactcctct 25740
tgccaacgcc aacctttgaa cacaccagtg tccctgcttt cccagggtcc taccctgagg 25800
gggaccctca tgatctggtc ctccctcttt ttccacactc atctgccatc aaacccgtcc 25860
ccgccgttcc accctgcagc ccccacccca gccacgcaga agggttcgca gttccatggg 25920

cgttccccgc ctccacaggg agccttccct gacccaccct gcccaccctc cccccccccc 25980
ccgctgggtt ggatcccccc cacctcctgg gggctctgtc gccctcccac tgtcagagcc 26040
ctcaggccac tgtagagaag cagcctcttt gccaacctgc tccctgccaa acagggcttc 26100
caagggacag ttatgcatgc tgcctggatc ccggcacctg gccctggcgc gagcctatgg 26160
cccacttaca catcagctga attcacacct gaccactgca gtttgttctc ttgaatttct 26220
tggaatttcc ttatggaggc tagttcactt ttgtaaacag aaaaagttgt atttttgcca 26280
taggtataaa gtaatattta ataaatgcag tggcaacaaa gcaaagctgt gttaaagtct 26340
gccagacact gttgcctaca gaaggtgcag caggaaggtg gctctctcct tgccaggaaa 26400
gagggagtag aaagtgtcag gggtgagaca cgggccagca ccatatggag attctcctga 26460
cagaagcaga agaaccaaaa gaactgaaag ggccgggtgc ggtggctcac acctgcaatc 26520
ccagcacttt gggaggccga ggtgggtgga tcacctgagg tcaggagctc gagaccaacc 26580
tagccaacat gatgaaaccc tgtctctacc caaaatacaa aaattagctg ggtgtggtgg 26640
tgcacgcctg taatcaggct actcgggagt ctgaggcagg agaatcgttt gaaccctgga 26700
ggtgaaggtt gcagtgagca gagattgcac cattgcactc cagcctgggc aacagagtga 26760
gactccgtct caaaagaaaa aaagaactga aaggagttct ctcacggaga ctaaatgtga 26820
ttacatcacc accttacatg cctccctggt ccgctgtggg gaacattgct ctaaagcgct 26880
caaagaggcg ttgttggatg ggactgtcat tgcttttatc cttcccatat gatcacctct 26940
ttttgaattt tgttttccag tacttcctgc cataaaactc cagataaagt aaaccatgca 27000
gtactggctg ttgggtatgg agaaaaaaat gggatccctt actggatcgt gaaaaactct 27060
tggggtcccc agtggggaat gaacgggtaa gcagctccca ggtcccactt ctgggagggg 27120
gatgttgctc aggcctggag gccctggggg catcacttcc acctcagagt tacggcttcc 27180
acagacgagc tggtcagcca agccctaggc caggccaggg ggaaagggag catgagaatg 27240
tagcctccac atttccaagc ccccatttgc agccctcaca gggccccagg tctttaggac 27300
gctgcaggga gagcaggagg ccagctggct cagaaggact gctaggaaaa cagctcagaa 27360
cttggaactc tgctggcacg aaatcagtgt ctgggagggt ccgtctttga gaaatcaaag 27420
caacaaatgt acccctgatc actgacagcc cctccttccg ggagaaaagg ctggcatggg 27480
gcggcctggg tgtatatgct gctgtgcctt tgtcctgtcc tcccagattc catgtccttg 27540
tctgaaaagg agagctttcc tgttccctcg ggctcctggt gataaaatag ctgtgtgagg 27600
gcccggccag ggctaagagg gggccctcgg taaattttcg ggagaagtgg gatatggatc 27660
attcatttgg cagctaagga gagggaagaa tgaattcttc acaaagtcag gtccgtgatt 27720
tgccggtgat caaataagcc gaatgctact gttttatgaa taaatttaga gggtctcacc 27780
cctgaagtca gctcttggtg agcagcaaaa cattctagaa aggcctgtgg tggggagacc 27840
gccggccggg cctcagctcc tctttctgcc caggtacttc ctcatcgagc gcggaaagaa 27900
catgtgtggc ctggctgcct gcgcctccta ccccatccct ctggtgtgag ccgtggcagc 27960

cgcagcgcag actggcggag aaggagagga acgggcagcc tgggcctggg tggaaatcct 28020
gccctggagg aagttgtggg gagatccact gggaccccca acattctgcc ctcacctctg 28080
tgcccagcct ggaaacctac agacaaggag gagttccacc atgagctcac ccgtgtctat 28140
gacgcaaaga tcaccagcca tgtgccttag tgtccttctt aacagactca aaccacatgg 28200
accacgaata ttctttctgt ccagaagggc tactttccac atatagagct ccagggactg 28260
tcttttctgt attcgctgtt caataaacat tgagtgagca cctccccaga tggagcatgc 28320
tggtcctggg ccatgactca aaccaggtct gtcctcgcca ctcagtctcc caggagggag 28380
gcatcagggg gtgtccaggc tctggctggg aggatggtgg gggtgctggc ggtattatcc 28440
accagatggg gtatccagga tgaagagcag gcttaatttg agatcttgac tatttgtccc 28500
aaacctgagt ggagaggtgg ggaggaagga gaaaaacggg ccaagctgcg gcagaggcag 28560
gattcaaacc cacaatgtca gcccagaagc ctgtacttct ctgtgtggcc tccccagtct 28620
cagctgccta ctcctactca gactctgccc cccacctcca catgagactg cccacccctt 28680
cctcaccaac cccttattta tttcaaacgt gcccagatgc tcctgtcgtt tccacaccat 28740
cccttccctt atcttgcctt ttgagaaatc ttgatctctg tttagagggc ggccatgaag 28800
ttgctgctgc ttcctgaagc ctccgggctc tgggatgtct gtagaacaag ctggagtcca 28860
ccagctcttt gtcctatggc tcacagactt tcttaatgat aaaagctgtc atcttatttg 28920
ggcttcctga aaagctaaag gcttgagaat atttgaaaaa tatatatgtt catgataagg 28980
cttatttaga taatgcaaat atagtttaat ttattagaaa attctagtaa cataagacca 29040
caactgtata agacaaaaaa caatcacctt gctaggcatg gtggctcacg cctataatcc 29100
cagcactttg ggaggccgag gcaggtggat cacctgaggt caggagtttg agactagcct 29160
ggccaacatg gtgaaacccc gtctctaata aaaatacaaa aaaaaaaatt tagccaggag 29220
tggtagtgca cacctgtagt cccagctact cgggagggtg aggcaagaga atcgcttaag 29280
cctgggagat ggaggttgca gtgagctgag atcatgccac tgcactccag cctcaaaaaa 29340
aaaataataa taataataga cttcattgga tggaattcta ttctagattt aaaaaaaaac 29400
taacatgaaa tgaaagcata atttcttttt ctttcttttt tttttttttt ttttagatgt 29460
agtctcactc tgtcacccag gctggagtgc agtggtgtga tctcagctca ctgcaacctc 29520
cacctcccca gttcaagcga ttctcctgcc tcagcctcct gaatagctgg gattacaggc 29580
atgcaccacc actcctggct aatttatgta tttttagtag agatggggtc tcaccatgtt 29640
ggtcaagctg gtctcgaact cctgacctca agtgatccca aagtgctggg attacaggag 29700
tgtgagcaac cgcacccagc ctgaaagcat aatttctgaa tttgacaaag attctcaaat 29760
caatggtcag ttgtataaaa ggattcccat tgaagtcagt ggtataaaag gattcccatt 29820
gaagatacat ggcaaataag tctaagggta ccattcttgc ttacatctat ctggaagatc 29880
tggctgctgt gataaataaa cccaattaat atttgcagac agcatgataa tgtggctaaa 29940
aacattcagg agaatcaaca aaacttagaa ctaatgaaag agttctgaaa gtggctggat 30000

accagaaaaa aaaatcaaaa actcagaaaa aatacactat tttttgttac tattgcaact 30060

ttactattgc aacaaaacca gcagctagga ataaccacca aaaagtctta tatgaagaaa 30120

attactaaat taataacact ataaaggaag acttagataa atggagagcc accatattct 30180

tttgttgttg ttgttgagac agagtctcac actgttgccc gggctggagt gcaatggcat 30240

gatcttggct cactgcaacc tccgcctccc gggttcacgt gattctcctg cctcagccac 30300

ccgagtagct gggattacag gtgcccacc 30329

<210> 2
<211> 1494
<212> DNA
<213> Homo sapiens

<400> 2
ccacgctcgt gccgctcccc ccccgcgctc ccagttgacg ctctgggccg ccacctccgc 60

ggaccctgag cgcaagagcc aagccgccag cgctgcgatg tgggccacgc tgccgctgct 120

ctgcgccggg gcctggctcc tgggagtccc cgtctgcggt gccgccgaac tgtgcgtgaa 180

ctccttagag aagtttcact tcaagtcatg gatgtctaag caccgtaaga cctacagtac 240

ggaggagtac caccacaggc tgcagacgtt tgccagcaac tggaggaaga taaacgccca 300

caacaatggg aaccacacat ttaaaatggc actgaaccaa ttttcagaca tgagctttgc 360

tgaaataaaa cacaagtatc tctggtcaga gcctcagaat tgctcagcca ccaaaagtaa 420

ctaccttcga ggtactggtc cctacccacc ttccgtggac tggcggaaaa aaggaaattt 480

tgtctcacct gtgaaaaatc agggtgcctg cggcagttgc tggactttct ccaccactgg 540

ggccctggag tctgcgatcg ccatcgcaac cggaaagatg ctgtccttgg cggaacagca 600

gctggtggac tgcgcccagg acttcaataa tcacggctgc caagggggtc tccccagcca 660

ggctttcgag tatatcctgt acaacaaggg gatcatgggt gaagacacct acccctacca 720

gggcaaggat ggttattgca agttccaacc tggaaaggcc atcggctttg tcaaggatgt 780

agccaacatc acaatctatg acgaggaagc gatggtggag gctgtggccc tctacaaccc 840

tgtgagcttt gcctttgagg tgactcagga cttcatgatg tatagaacgg gcatctactc 900

cagtacttcc tgccataaaa ctccagataa agtaaaccat gcagtactgg ctgttgggta 960

tggagaaaaa aatgggatcc cttactggat cgtgaaaaac tcttggggtc cccagtgggg 1020

aatgaacggg tacttcctca tcgagcgcgg aaagaacatg tgtggcctgg ctgcctgcgc 1080

ctcctacccc atccctctgg tgtgagccgt ggcagccgca gcgcagactg gcggagaagg 1140

agaggaacgg gcagcctggg cctgggtgga aatcctgccc tggaggaagt tgtggggaga 1200

tccactggga cccccaacat tctgccctca cctctgtgcc cagcctggaa acctacagac 1260

aaggaggagt tccaccatga gctcacccgt gtctatgacg caaagatcac cagccatgtg 1320

ccttagtgtc cttcttaaca gactcaaacc acatggacca cgaatattct ttctgtccag 1380

aagggctact ttccacatat agagctccag ggactgtctt ttctgtattc gctgttcaat 1440

aaacattgag tgagcacctc cccagatgga gcatgctggt cctggaaaaa aaaa 1494

<210> 3
<211> 1458
<212> DNA
<213> Homo sapiens

<400> 3
ccacgctcgt gccgctcccc ccccgcgctc ccagttgacg ctctgggccg ccacctccgc 60

ggaccctgag cgcaagagcc aagccgccag cgctgcgatg tgggccacgc tgccgctgct 120

ctgcgccgcc gaactgtgcg tgaactcctt agagaagttt cacttcaagt catggatgtc 180

taagcaccgt aagacctaca gtacggagga gtaccaccac aggctgcaga cgtttgccag 240

caactggagg aagataaacg cccacaacaa tgggaaccac acatttaaaa tggcactgaa 300

ccaattttca gacatgagct ttgctgaaat aaaacacaag tatctctggt cagagcctca 360

gaattgctca gccaccaaaa gtaactacct tcgaggtact ggtccctacc caccttccgt 420

ggactggcgg aaaaaaggaa attttgtctc acctgtgaaa aatcagggtg cctgcggcag 480

ttgctggact ttctccacca ctggggccct ggagtctgcg atcgccatcg caaccggaaa 540

gatgctgtcc ttggcggaac agcagctggt ggactgcgcc caggacttca ataatcacgg 600

ctgccaaggg ggtctcccca gccaggcttt cgagtatatc ctgtacaaca aggggatcat 660

gggtgaagac acctacccct accagggcaa ggatggttat tgcaagttcc aacctggaaa 720

ggccatcggc tttgtcaagg atgtagccaa catcacaatc tatgacgagg aagcgatggt 780

ggaggctgtg gccctctaca accctgtgag ctttgccttt gaggtgactc aggacttcat 840

gatgtataga acgggcatct actccagtac ttcctgccat aaaactccag ataaagtaaa 900

ccatgcagta ctggctgttg ggtatggaga aaaaaatggg atcccttact ggatcgtgaa 960

aaactcttgg ggtccccagt ggggaatgaa cgggtacttc ctcatcgagc gcggaaagaa 1020

catgtgtggc ctggctgcct gcgcctccta ccccatccct ctggtgtgag ccgtggcagc 1080

cgcagcgcag actggcggag aaggagagga acgggcagcc tgggcctggg tggaaatcct 1140

gccctggagg aagttgtggg gagatccact gggaccccca acattctgcc ctcacctctg 1200

tgcccagcct ggaaacctac agacaaggag gagttccacc atgagctcac ccgtgtctat 1260

gacgcaaaga tcaccagcca tgtgccttag tgtccttctt aacagactca aaccacatgg 1320

accacgaata ttctttctgt ccagaagggc tactttccac atatagagct ccagggactg 1380

tcttttctgt attcgctgtt caataaacat tgagtgagca cctccccaga tggagcatgc 1440

tggtcctgga aaaaaaaa 1458

<210> 4
<211> 1372
<212> DNA
<213> Mus musculus

<400> 4
gggaccgggc agtgagcgcc gagatgtggg ctgcgctgcc gctgctgtgc gctggggcct 60

ggctgctgag tactggggcc accgccgagc tgaccgtgaa cgccatagaa aagtttcact 120
ttaagtcatg gatgaaacag catcaaaaga cgtacagctc ggtggagtac aaccacagac 180
tgcagatgtt tgccaacaac tggaggaaga ttcaagccca caaccagagg aaccacacat 240
ttaaaatggc attgaaccag ttttcagata tgagctttgc tgaaataaaa cacaaattcc 300
tttggtcaga gcctcagaat tgctcagcca ccaaaagtaa ctacctccga ggtacaggcc 360
cctacccttc ctccatggac tggaggaaga aaggaaatgt tgtttcgcca gtgataaatc 420
agggggcctg tggcagctgc tggactttct ctaccacggg ggccctagag tcagctgtgg 480
ctattgccag tgggaaaatg ctgtctttgg ctgagcagca gctggtggat tgtgcccaag 540
ccttcaacaa tcatggctgc aaaggaggtc tccccagcca ggccttcgag tacatcctat 600
acaacaaggg catcatggaa gaagacagct acccttacat aggcaaggat agttcatgca 660
gattcaaccc ccaaaaagct gttgcattcg tcaagaatgt tgtcaacatc acactcaatg 720
acgaggctgc aatggttgag gctgtggctc tatacaaccc tgtgagcttc gcctttgagg 780
tgactgaaga ttttttgatg tataaaagtg gcgtctactc cagtaaatcc tgtcataaaa 840
ctccagataa agtaaaccat gcagtcctgg cggttggcta tggagaacag aatggattac 900
tctactggat tgtgaaaaac tcttggggct cccagtgggg ggagaatggg tacttcctca 960
ttgaacgtgg gaagaacatg tgtggcctgg ctgcttgtgc ctcctatccc attcctcagg 1020
tataagccac ggctgcacgg gcaactgatt ggcagaccaa gggaggaact ggtccttcga 1080
tgagaatgcc accctggaga aaattgtgtg gaaatccacc cagaggccct ctcactcctg 1140
agtctagacg cctaaagaca ggaaggacaa acttgaatag cgacaagccc acccacgtga 1200
catcatcacc agaaatacgc ttggattgtg gttttttaat gacccaaacc cacgtggacc 1260
tagaatcgtc tctctttcca gctctcttca tgtactggga gctgtaatgg ttaccttttc 1320
tatgttgtgt attcaataaa cacacagtaa atacctcaaa aaaaaaaaaa aa 1372

<210> 5
<211> 335
<212> PRT
<213> Homo sapiens

<400> 5

<210>  6
<211>  335
<212>  PRT
<213>  Homo sapiens

<400>  6

Met Trp Ala Thr Leu Pro Leu Leu Cys Ala Gly Ala Trp Leu Leu Gly
1               5                   10                  15

Val Pro Val Cys Gly Ala Ala Glu Leu Cys Val Asn Ser Leu Glu Lys
            20                  25                  30

Phe His Phe Lys Ser Trp Met Ser Lys His Arg Lys Thr Tyr Ser Thr
        35                  40                  45

Glu Glu Tyr His His Arg Leu Gln Thr Phe Ala Ser Asn Trp Arg Lys
    50                  55                  60

Ile Asn Ala His Asn Asn Gly Asn His Thr Phe Lys Met Ala Leu Asn
65                  70                  75                  80

Gln Phe Ser Asp Met Ser Phe Ala Glu Ile Lys His Lys Tyr Leu Trp
                85                  90                  95

Ser Glu Pro Gln Asn Cys Ser Ala Thr Lys Ser Asn Tyr Leu Arg Gly
            100                 105                 110

Thr Gly Pro Tyr Pro Pro Ser Val Asp Trp Arg Lys Lys Gly Asn Phe
        115                 120                 125

Val Ser Pro Val Lys Asn Gln Gly Ala Cys Gly Ser Cys Trp Thr Phe
    130                 135                 140

Ser Thr Thr Gly Ala Leu Glu Ser Ala Ile Ala Ile Ala Thr Gly Lys
145                 150                 155                 160

Met Leu Ser Leu Ala Glu Gln Gln Leu Val Asp Cys Ala Gln Asp Phe
                165                 170                 175

Asn Asn His Gly Cys Gln Gly Gly Leu Pro Ser Gln Ala Phe Glu Tyr
            180                 185                 190

Ile Leu Tyr Asn Lys Gly Ile Met Gly Glu Asp Thr Tyr Pro Tyr Gln
        195                 200                 205

Gly Lys Asp Gly Tyr Cys Lys Phe Gln Pro Gly Lys Ala Ile Gly Phe
    210                 215                 220

Val Lys Asp Val Ala Asn Ile Thr Ile Tyr Asp Glu Glu Ala Met Val
225                 230                 235                 240

Glu Ala Val Ala Leu Tyr Asn Pro Val Ser Phe Ala Phe Glu Val Thr

245                 250                 255

Gln Asp Phe Met Met Tyr Arg Thr Gly Ile Tyr Ser Ser Thr Ser Cys
            260                 265                 270

His Lys Thr Pro Asp Lys Val Asn His Ala Val Leu Ala Val Gly Tyr
        275                 280                 285

Gly Glu Lys Asn Gly Ile Pro Tyr Trp Ile Val Lys Asn Ser Trp Gly
    290                 295                 300

Pro Gln Trp Gly Met Asn Gly Tyr Phe Leu Ile Glu Arg Gly Lys Asn
305                 310                 315                 320

Met Cys Gly Leu Ala Ala Cys Ala Ser Tyr Pro Ile Pro Leu Val
                325                 330                 335

<210>  7
<211>  323
<212>  PRT
<213>  Homo sapiens

<400>  7

Met Trp Ala Thr Leu Pro Leu Leu Cys Ala Ala Glu Leu Cys Val Asn
1               5                   10                  15

Ser Leu Glu Lys Phe His Phe Lys Ser Trp Met Ser Lys His Arg Lys
            20                  25                  30

Thr Tyr Ser Thr Glu Glu Tyr His His Arg Leu Gln Thr Phe Ala Ser
        35                  40                  45

Asn Trp Arg Lys Ile Asn Ala His Asn Asn Gly Asn His Thr Phe Lys
    50                  55                  60

Met Ala Leu Asn Gln Phe Ser Asp Met Ser Phe Ala Glu Ile Lys His
65                  70                  75                  80

Lys Tyr Leu Trp Ser Glu Pro Gln Asn Cys Ser Ala Thr Lys Ser Asn
                85                  90                  95

Tyr Leu Arg Gly Thr Gly Pro Tyr Pro Pro Ser Val Asp Trp Arg Lys
            100                 105                 110

Lys Gly Asn Phe Val Ser Pro Val Lys Asn Gln Gly Ala Cys Gly Ser
        115                 120                 125

Cys Trp Thr Phe Ser Thr Thr Gly Ala Leu Glu Ser Ala Ile Ala Ile
    130                 135                 140

Ala Thr Gly Lys Met Leu Ser Leu Ala Glu Gln Gln Leu Val Asp Cys
145                 150                 155                 160

Ala Gln Asp Phe Asn Asn His Gly Cys Gln Gly Gly Leu Pro Ser Gln
                165                 170                 175

Ala Phe Glu Tyr Ile Leu Tyr Asn Lys Gly Ile Met Gly Glu Asp Thr
            180                 185                 190

Tyr Pro Tyr Gln Gly Lys Asp Gly Tyr Cys Lys Phe Gln Pro Gly Lys
        195                 200                 205

Ala Ile Gly Phe Val Lys Asp Val Ala Asn Ile Thr Ile Tyr Asp Glu
    210                 215                 220

Glu Ala Met Val Glu Ala Val Ala Leu Tyr Asn Pro Val Ser Phe Ala
225                 230                 235                 240

Phe Glu Val Thr Gln Asp Phe Met Met Tyr Arg Thr Gly Ile Tyr Ser
                245                 250                 255

Ser Thr Ser Cys His Lys Thr Pro Asp Lys Val Asn His Ala Val Leu
            260                 265                 270

Ala Val Gly Tyr Gly Glu Lys Asn Gly Ile Pro Tyr Trp Ile Val Lys
        275                 280                 285

Asn Ser Trp Gly Pro Gln Trp Gly Met Asn Gly Tyr Phe Leu Ile Glu
    290                 295                 300

Arg Gly Lys Asn Met Cys Gly Leu Ala Ala Cys Ala Ser Tyr Pro Ile
305                 310                 315                 320

Pro Leu Val

<210>  8
<211>  323
<212>  PRT
<213>  Homo sapiens

<400>  8

Met Trp Ala Thr Leu Pro Leu Leu Cys Ala Ala Glu Leu Cys Val Asn
1               5                   10                  15

Ser Leu Glu Lys Phe His Phe Lys Ser Trp Met Ser Lys His Arg Lys
            20                  25                  30

Thr Tyr Ser Thr Glu Glu Tyr His His Arg Leu Gln Thr Phe Ala Ser
        35                  40                  45

Asn Trp Arg Lys Ile Asn Ala His Asn Asn Gly Asn His Thr Phe Lys
    50                  55                  60

Met Ala Leu Asn Gln Phe Ser Asp Met Ser Phe Ala Glu Ile Lys His
65                  70                  75                  80

Lys Tyr Leu Trp Ser Glu Pro Gln Asn Cys Ser Ala Thr Lys Ser Asn
                85                  90                  95

Tyr Leu Arg Gly Thr Gly Pro Tyr Pro Pro Ser Val Asp Trp Arg Lys
            100                 105                 110

Lys Gly Asn Phe Val Ser Pro Val Lys Asn Gln Gly Ala Cys Gly Ser
        115                 120                 125

Cys Trp Thr Phe Ser Thr Thr Gly Ala Leu Glu Ser Ala Ile Ala Ile
    130                 135                 140

Ala Thr Gly Lys Met Leu Ser Leu Ala Glu Gln Gln Leu Val Asp Cys
145                 150                 155                 160

Ala Gln Asp Phe Asn Asn His Gly Cys Gln Gly Gly Leu Pro Ser Gln
                165                 170                 175

Ala Phe Glu Tyr Ile Leu Tyr Asn Lys Gly Ile Met Gly Glu Asp Thr
            180                 185                 190

Tyr Pro Tyr Gln Gly Lys Asp Gly Tyr Cys Lys Phe Gln Pro Gly Lys
        195                 200                 205

Ala Ile Gly Phe Val Lys Asp Val Ala Asn Ile Thr Ile Tyr Asp Glu
    210                 215                 220

Glu Ala Met Val Glu Ala Val Ala Leu Tyr Asn Pro Val Ser Phe Ala
225                 230                 235                 240

Phe Glu Val Thr Gln Asp Phe Met Met Tyr Arg Thr Gly Ile Tyr Ser
                245                 250                 255

Ser Thr Ser Cys His Lys Thr Pro Asp Lys Val Asn His Ala Val Leu
            260                 265                 270

Ala Val Gly Tyr Gly Glu Lys Asn Gly Ile Pro Tyr Trp Ile Val Lys
        275                 280                 285

Asn Ser Trp Gly Pro Gln Trp Gly Met Asn Gly Tyr Phe Leu Ile Glu
    290                 295                 300

Arg Gly Lys Asn Met Cys Gly Leu Ala Ala Cys Ala Ser Tyr Pro Ile
305                 310                 315                 320

Pro Leu Val

<210>  9
<211>  334
<212>  PRT
<213>  Homo sapiens

<400>  9

Met Trp Ala Thr Leu Pro Leu Leu Cys Ala Gly Ala Trp Leu Leu Gly
1               5                   10                  15

Val Pro Val Cys Gly Ala Ala Glu Leu Cys Val Asn Ser Leu Glu Lys
            20                  25                  30

Phe His Phe Lys Ser Trp Met Ser Lys His Arg Lys Thr Tyr Ser Thr
        35                  40                  45

Glu Glu Tyr His His Arg Leu Gln Thr Phe Ala Ser Asn Trp Arg Lys
    50                  55                  60

Ile Asn Ala His Asn Asn Gly Asn His Thr Phe Lys Met Ala Leu Asn
65                  70                  75                  80

Gln Phe Ser Asp Met Ser Phe Ala Glu Ile Lys His Lys Tyr Leu Trp
                85                  90                  95

Ser Glu Pro Gln Asn Cys Ser Ala Thr Lys Ser Asn Tyr Leu Arg Gly
            100                 105                 110

Thr Gly Pro Tyr Pro Pro Ser Val Asp Trp Arg Lys Lys Gly Asn Phe
        115                 120                 125

Val Ser Pro Val Lys Asn Gln Gly Ala Cys Gly Ser Cys Trp Thr Phe
    130                 135                 140

Ser Thr Thr Gly Ala Leu Glu Ser Ala Ile Ala Ile Ala Thr Gly Lys
145                 150                 155                 160

Met Leu Ser Leu Ala Glu Gln Gln Leu Val Asp Cys Ala Gln Asp Phe
                165                 170                 175

Asn Asn His Gly Cys Gln Gly Gly Leu Pro Ser Gln Ala Phe Glu Tyr
            180                 185                 190

Ile Leu Tyr Asn Lys Gly Ile Met Gly Glu Asp Thr Tyr Pro Tyr Gln
        195                 200                 205

Gly Lys Asp Gly Tyr Cys Lys Phe Gln Pro Gly Lys Ala Ile Gly Phe
    210                 215                 220

Val Lys Asp Val Ala Asn Ile Thr Ile Tyr Asp Glu Glu Ala Met Val
225                 230                 235                 240

Glu Ala Val Ala Leu Tyr Asn Pro Val Ser Phe Ala Phe Glu Val Thr
                245                 250                 255

Gln Asp Phe Met Met Tyr Arg Thr Gly Ile Tyr Ser Ser Thr Ser Cys
            260                 265                 270

His Lys Thr Pro Asp Lys Val Asn His Ala Val Leu Ala Val Gly Tyr
        275                 280                 285

Gly Glu Lys Asn Gly Ile Pro Tyr Trp Ile Val Lys Asn Ser Trp Gly
    290                 295                 300

Pro Gln Trp Gly Met Asn Gly Tyr Phe Leu Ile Glu Arg Gly Lys Asn
305                 310                 315                 320

Met Cys Gly Leu Ala Ala Cys Ala Ser Tyr Pro Ile Pro Leu
                325                 330

<210>  10
<211>  20
<212>  DNA
<213>  Artificial

<220>
<223>  Forward Primer

<400>  10
gcgctcccag ttgacgctct                                                   20

<210>  11
<211>  18
<212>  DNA
<213>  Artificial

<220>
<223>  Reverse primer

<400>  11
cacgcacagt tcggcggc                                                     18

<210>  12
<211>  21
<212>  DNA
<213>  Artificial

<220>
<223>  Forward primer

<400>  12
cgctcccagt tgacgctctg g                                                 21

<210>  13
<211>  18
<212>  DNA
<213>  Artificial

<220>
<223>  Reverse primer

<400>  13
cacgcacagt tcggcggc                                                    18


<210>  14
<211>  39
<212>  DNA
<213>  Artificial

<220>
<223>

<400>  14
ggccagtgaa ttgtaatacg actcactata gggaggcgg                             39